(19) **Europäisches Patentamt European Patent Office Office européen des brevets**

(11) **EP 3 517 043 B1**

(12) **EUROPEAN PATENT SPECIFICATION**

(45) Date of publication and mention
of the grant of the patent:
**24.05.2023 Bulletin 2023/21**

(51) International Patent Classification (IPC):
**A61B 8/08** (2006.01)     **A61B 8/00** (2006.01)
**A61B 8/06** (2006.01)

(21) Application number: **17853307.1**

(22) Date of filing: **24.08.2017**

(52) Cooperative Patent Classification (CPC):
**A61B 8/488; A61B 8/065; A61B 8/0891;**
**A61B 8/4405; A61B 8/461; A61B 8/5207;**
**G01S 7/52028; G01S 7/52077; G01S 15/8979;**
A61B 8/06; A61B 8/08; A61B 8/4427; A61B 8/4472;
A61B 8/463; A61B 8/464;     (Cont.)

(86) International application number:
**PCT/KR2017/009248**

(87) International publication number:
**WO 2018/056593 (29.03.2018 Gazette 2018/13)**

(54) **ULTRASONIC IMAGING DEVICE AND ULTRASONIC IMAGE DISPLAY METHOD**

ULTRASCHALLBILDGEBUNGSVORRICHTUNG UND ULTRASCHALLBILDANZEIGEVERFAHREN

DISPOSITIF D'IMAGERIE ULTRASONORE ET PROCÉDÉ D'AFFICHAGE D'IMAGE ULTRASONORE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.09.2016 US 201662396910 P**
**20.09.2016 US 201662396970 P**
**20.09.2016 US 201662396889 P**
**09.06.2017 KR 20170072725**
**09.06.2017 KR 20170072726**
**09.06.2017 KR 20170072727**

(43) Date of publication of application:
**31.07.2019 Bulletin 2019/31**

(73) Proprietors:
• **Samsung Medison Co., Ltd.**
**Hongcheon-gun, Gangwon-do 25108 (KR)**
• **Sogang University Research Foundation**
**Seoul 04107 (KR)**

(72) Inventors:
• **LEE, Woo-youl**
**Hongcheon-gun**
**Gangwon-do 25108 (KR)**
• **YANG, Eun-ho**
**Hongcheon-gun**
**Gangwon-do 25108 (KR)**
• **CHO, Gae-young**
**Hongcheon-gun**
**Gangwon-do 25108 (KR)**
• **KANG, Jin-bum**
**Seoul 02794 (KR)**
• **YOO, Yang-mo**
**Seoul 07214 (KR)**

(74) Representative: **Arnold & Siedsma**
**Bezuidenhoutseweg 57**
**2594 AC The Hague (NL)**

(56) References cited:
WO-A1-2007/107926      JP-A- 2003 079 625
JP-A- 2006 141 996       KR-A- 20080 084 442
KR-A- 20100 043 686      KR-A- 20160 094 221
US-A1- 2003 163 044      US-A1- 2010 286 523
US-A1- 2013 006 111      US-A1- 2013 172 754
US-A1- 2014 018 680      US-A1- 2016 128 673
US-A1- 2016 220 231

(52) Cooperative Patent Classification (CPC): (Cont.)
A61B 8/467; A61B 8/5223; A61B 8/5269;
A61B 8/5276

**Description**

TECHNICAL FIELD

The present disclosure relates to an ultrasonic imaging apparatus for generating and displaying a spectral Doppler signal and a method of displaying an ultrasound image.

BACKGROUND ART

Ultrasonic imaging apparatuses generate ultrasound signals for a predetermined inner region of an object via a probe and use information of reflected echo signals to obtain an image of the inner region of the object. In particular, ultrasonic imaging apparatuses are used for medical purposes including observation, assessment of injuries, and detection of foreign substances inside an object. Such ultrasonic imaging apparatuses exhibit high stability, display images in real time, and are safe due to lack of radiation exposure compared to diagnostic X-ray apparatuses. Therefore, ultrasonic imaging apparatuses have been widely used together with other types of imaging diagnosis apparatuses.

Furthermore, ultrasonic imaging apparatuses may provide a Doppler image showing movement of an object to a user. In other words, ultrasonic imaging apparatuses may measure and output movement of the object by using various Doppler images such as color Doppler, spectral Doppler, and vector Doppler images.

As prior art is known US 2003/163044 showing a method and an apparatus in a diagnostic ultra-sound system for generating and displaying strain rate spectrums corresponding to the deformation of a tissue structure within a subject, designated by a sample rate, in response to Doppler signals generated by the ultrasound system.

Further, document US2013/006111 discloses an apparatus placing two range gates on a Region of Interest (ROI) for interrogating via Doppler ultrasound a first and a second motion of a living body. The first motion can be blood flow and the second tissue movement or lymph flow. Alternatively, the first motion can be blood flow in an artery and the second motion blood flow in a vein.

DESCRIPTION OF EMBODIMENTS

TECHNICAL PROBLEM

Provided are an ultrasonic imaging apparatus for generating and displaying a spectral Doppler signal and a method of displaying an ultrasound image.

SOLUTION TO PROBLEM

According to an aspect of the present disclosure, an ultrasonic imaging apparatus includes features of the appended independent apparatus claim. The present disclosure also relates to a corresponding method.

ADVANTAGEOUS EFFECTS OF DISCLOSURE

According to some embodiments, an ultrasonic imaging apparatus may divide a Doppler signal corresponding to a sample volume into a plurality of sub-Doppler signals and generate a spectral Doppler signal corresponding to the sample volume based on a plurality of spectral Doppler signals respectively corresponding to the plurality of sub-Doppler signals. Thus, it is possible to display the spectral Doppler signal corresponding to the sample volume as a high-quality image.

BRIEF DESCRIPTION OF DRAWINGS

FIG. 1 is a block diagram of a configuration of an ultrasound diagnosis apparatus according to an embodiment.
FIGS. 2A through 2C illustrate ultrasound diagnosis apparatuses according to embodiments.
FIG. 3 is a block diagram of an ultrasonic imaging apparatus according to an embodiment.
FIG. 4 is a diagram illustrating an embodiment in which an ultrasonic imaging apparatus generates a spectral Doppler signal corresponding to a sample volume.
FIG. 5 is a diagram illustrating a detailed embodiment in which an ultrasonic imaging apparatus generates a spectral Doppler signal corresponding to a sample volume.
FIG. 6 is a diagram illustrating an embodiment in which an ultrasonic apparatus generates a spectral Doppler signal corresponding to a sample volume.

FIG. 7 is a flowchart of a method by which an ultrasonic imaging apparatus displays an ultrasound image, according to an embodiment.

FIG. 8 is a block diagram of an ultrasonic imaging apparatus according to another embodiment.

FIG. 9 illustrates an embodiment in which an ultrasonic imaging apparatus displays a plurality of spectral Doppler signals.

FIG. 10 illustrates an embodiment in which an ultrasonic imaging apparatus displays a plurality of spectral Doppler signals as a single image.

FIG. 11 illustrates an embodiment in which an ultrasonic imaging apparatus displays a plurality of spectral Doppler signals.

FIG. 12 illustrates an embodiment in which an ultrasonic imaging apparatus provides information about vascular wall stiffness.

FIG. 13 illustrates an embodiment in which an ultrasonic imaging apparatus displays a plurality of spectral Doppler signals.

FIG. 14 illustrates a method by which an ultrasonic imaging apparatus displays an ultrasound image, according to an embodiment.

FIG. 15 is a block diagram of an ultrasonic imaging apparatus according to another embodiment.

FIG. 16 illustrates an embodiment in which an ultrasonic imaging apparatus generates a spectral Doppler signal corresponding to an object.

FIG. 17 illustrates a detailed embodiment in which an ultrasonic imaging apparatus generates a spectral Doppler signal corresponding to an object.

FIG. 18 illustrates another embodiment in which an ultrasonic imaging apparatus generates a spectral Doppler signal corresponding to an object.

FIG. 19 is a flowchart of a method by which an ultrasonic imaging apparatus displays an ultrasound image, according to an embodiment.

BEST MODE

According to an embodiment, an ultrasonic imaging apparatus may include: a processor configured to acquire a Doppler signal corresponding to a sample volume, divide the Doppler signal into a plurality of sub-Doppler signals, generate a plurality of spectral Doppler signals by performing spectral analysis on each of the plurality of sub-Doppler signals, and generate, from the plurality of spectral Doppler signals, a spectral Doppler signal corresponding to the sample volume; and a display displaying the spectral Doppler signal corresponding to the sample volume.

According to an embodiment, the processor may generate the plurality of spectral Doppler signals by performing weighting accumulation and Fourier Transform on each of the plurality of sub-Doppler signals.

According to an embodiment, the processor may perform weighting accumulation by using a predetermined window function.

According to an embodiment, the processor may acquire the Doppler signal every period according to a pulse repetition frequency (PRF).

According to an embodiment, the processor may generate the spectral Doppler signal corresponding to the sample volume by performing weighting accumulation on the plurality of spectral Doppler signals.

According to the invention, the processor is configured to divide the Doppler signal into the plurality of sub-Doppler signals respectively corresponding to a plurality of sections set in the sample volume.

According to an embodiment, the number and length of the plurality of sections may be set based on a user input.

According to an embodiment, the processor may determine at least one effective spectral Doppler signal from among the plurality of spectral Doppler signals and generate the spectral Doppler signal corresponding to the sample volume from the determined at least one spectral Doppler signal.

According to an embodiment, the ultrasonic imaging apparatus may further include an ultrasound transceiver configured to control a probe to transmit a generated ultrasound signal to the sample volume as many times as a predetermined ensemble number and according to a preset PRF, and receive an echo signal reflected from the sample volume, and the processor may acquire the Doppler signal based on the echo signal.

According to an embodiment, a method of displaying an ultrasound image may include: acquiring a Doppler signal corresponding to a sample volume; dividing the Doppler signal into a plurality of sub-Doppler signals; generating a plurality of spectral Doppler signals by performing spectral analysis on each of the plurality of sub-Doppler signals; generating, from the plurality of spectral Doppler signals, a spectral Doppler signal corresponding to the sample volume; and displaying the spectral Doppler signal corresponding to the sample volume.

According to an embodiment, an ultrasonic imaging apparatus may include: a processor configured to acquire a Doppler signal corresponding to a sample volume, divide the Doppler signal into a plurality of sub-Doppler signals respectively corresponding to a plurality of sections set in the sample volume, and generate a plurality of spectral Doppler signals respectively corresponding to the plurality of sections by performing spectral analysis on each of the plurality of sub-Doppler signals; and a display displaying the plurality of spectral Doppler signals.

According to an embodiment, the processor may generate a plurality of spectral lines respectively corresponding to a plurality of sections by performing line tracing on the respective plurality of spectral Doppler signals, and the display may display the plurality of spectral lines.

According to an embodiment, when the sample volume is set with respect to a blood vessel, the processor may generate information about vascular wall stiffness based on the plurality of spectral Doppler signals, and the display may display the information about vascular wall stiffness.

According to an embodiment, the processor may respectively detect peak systolic velocities (PSVs) for the plurality of sections based on the plurality of spectral Doppler signals and generate information about vascular wall stiffness based on the PSVs for the plurality of sections.

According to an embodiment, when the sample volume is set with respect to an object, the processor may select at least one spectral Doppler signal representing the object from among the plurality of spectral Doppler signals, and the display may display the selected at least one spectral Doppler signal.

According to an embodiment, the ultrasonic imaging apparatus may further include a user input interface that receives an input of selecting at least one spectral Doppler signal representing the object from a user.

According to an embodiment, the object may be a microscopic blood vessel.

According to an embodiment, the number and length of the plurality of sections may be set based on a user input.

According to an embodiment, the processor may generate the plurality of spectral Doppler signals by respectively performing weighting accumulation and Fourier Transformation on the plurality of sub-Doppler signals.

According to an embodiment, a method of displaying an ultrasound image may include: acquiring a Doppler signal corresponding to a sample volume; dividing the Doppler signal into a plurality of sub-Doppler signals respectively corresponding to a plurality of sections set in the sample volume; generating a plurality of spectral Doppler signals respectively corresponding to the plurality of sections by performing spectral analysis on each of the plurality of sub-Doppler signals; and displaying the plurality of spectral Doppler signals.

According to an embodiment, an ultrasonic imaging apparatus may include: a processor configured to acquire a Doppler signal corresponding to a sample volume set with respect to an object, generate a plurality of spectral Doppler signals respectively corresponding to a plurality of sections set in the sample volume by performing spectral analysis on each of the Doppler signal from the plurality of sections, model an artifact signal in a spectral Doppler signal corresponding to the object by using the plurality of spectral Doppler signals, and generate, based on the spectral Doppler signal corresponding to the object and the artifact signal, a spectral Doppler signal corresponding to the object and from which an artifact is removed; and a display displaying the spectral Doppler signal corresponding to the object and from which the artifact is removed.

According to an embodiment, the processor may determine a spectral Doppler signal corresponding to a section including the object from among the plurality of sections as the spectral Doppler signal corresponding to the object.

According to the invention, the processor is configured to determine as an artifact signal an ultrasound reflected signal from another object, which is contained in the spectral Doppler signal corresponding to the object and model the artifact signal by using a spectral Doppler signal corresponding to a section including the other object from among the plurality of sections.

According to an embodiment, the object may correspond to a vein while the other object may correspond to an artery.

According to the invention, the processor is configured to generate the spectral Doppler signal corresponding to the object and from which the artifact is removed by performing subtraction between the spectral Doppler signal corresponding to the object and the artifact signal.

According to an embodiment, the processor may divide the Doppler signal into a plurality of sub-Doppler signals respectively corresponding to the plurality of sections and generate the plurality of spectral Doppler signals by performing spectral analysis on each of the plurality of sub-Doppler signals.

According to an embodiment, the ultrasonic imaging apparatus may further include an ultrasound transceiver configured to control a probe to transmit ultrasound signals along a pulsed Doppler line passing through the sample volume set with respect to the object and receive echo signals reflected from the sample volume and at least one volume on the pulsed Doppler line. The processor may acquire from the received echo signals a Doppler signal corresponding to the sample volume and at least one Doppler signal corresponding to the at least one volume, generate a spectral Doppler signal corresponding to the object and at least one spectral Doppler signal by performing spectral analysis on each of the Doppler signal corresponding to the sample volume and the at least one Doppler signal, model an artifact signal in the spectral Doppler signal corresponding to the object by using the at least one spectral Doppler signal, and generate, based on the spectral Doppler signal corresponding to the object and the artifact signal, a spectral Doppler signal corresponding to the object and from which an artifact is removed.

According to an embodiment, a method of displaying an ultrasound image may include: acquiring a Doppler signal corresponding to a sample volume set with respect to an object; generating a plurality of spectral Doppler signals respectively corresponding to a plurality of sections set in the sample volume by performing spectral analysis on the Doppler signal in each of the plurality of sections; modeling an artifact signal in a spectral Doppler signal corresponding to the object by using the plurality of spectral Doppler signals; generating, based on the spectral Doppler signal corresponding to the object and the artifact signal, a spectral Doppler signal corresponding to the object and from which an artifact is removed; and displaying the spectral Doppler signal corresponding to the object and from which the artifact is removed.

MODE OF DISCLOSURE

The present specification describes principles of the present disclosure and sets forth embodiments thereof to clarify the scope of the present disclosure and to allow those of ordinary skill in the art to implement the embodiments. The present embodiments may have different forms.

Like reference numerals refer to like elements throughout. The present specification does not describe all components in the embodiments, and common knowledge in the art or the same descriptions of the embodiments will be omitted below. The term "part" or "portion" may be implemented using hardware or software, and according to embodiments, one "part" or "portion" may be formed as a single unit or element or include a plurality of units or elements. Hereinafter, the principles and embodiments of the present disclosure will be described in detail with reference to the accompanying drawings.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ, blood vessel or a tissue, or a phantom.

Throughout the specification, an ultrasound image refers to an image of an object processed based on ultrasound signals transmitted to the object and reflected therefrom.

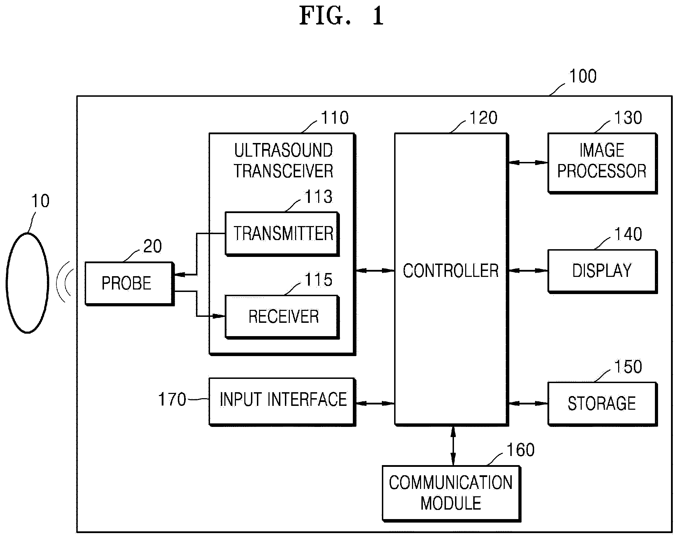

FIG. 1 is a block diagram illustrating a configuration of an ultrasound diagnosis apparatus 100, i.e., a diagnostic apparatus, according to an exemplary embodiment.

Referring to FIG. 1, the ultrasound diagnosis apparatus 100 may include a probe 20, an ultrasound transceiver 110, a controller 120, an image processor 130, one or more displays 140, a storage 150, e.g., a memory, a communication module 160, i.e., a communication device or an interface, and an input interface 170.

The ultrasound diagnosis apparatus 100 may be of a cart-type or a portable-type ultrasound diagnosis apparatus, that is portable, moveable, mobile, or hand-held. Examples of the portable-type ultrasound diagnosis apparatus 100 may include a smart phone, a laptop computer, a personal digital assistant (PDA), and a tablet personal computer (PC), each of which may include a probe and a software application, but embodiments are not limited thereto.

The probe 20 may include a plurality of transducers. The plurality of transducers may transmit ultrasound signals to an object 10 in response to transmitting signals received by the probe 20, from a transmitter 113. The plurality of transducers may receive ultrasound signals reflected from the object 10 to generate reception signals. In addition, the probe 20 and the ultrasound diagnosis apparatus 100 may be formed in one body (e.g., disposed in a single housing), or the probe 20 and the ultrasound diagnosis apparatus 100 may be formed separately (e.g., disposed separately in separate housings) but linked wirelessly or via wires. In addition, the ultrasound diagnosis apparatus 100 may include one or more probes 20 according to embodiments.

The controller 120 may control the transmitter 113 for the transmitter 113 to generate transmitting signals to be applied to each of the plurality of transducers based on a position and a focal point of the plurality of transducers included in the probe 20.

The controller 120 may control the ultrasound receiver 115 to generate ultrasound data by converting reception signals received from the probe 20 from analogue to digital signals and summing the reception signals converted into digital form, based on a position and a focal point of the plurality of transducers.

The image processor 130 may generate an ultrasound image by using ultrasound data generated from the ultrasound receiver 115. The ultrasound image may be not only a grayscale ultrasound image obtained by scanning an object in an amplitude (A) mode, a brightness (B) mode, and a motion (M) mode, but also a Doppler image representing a moving object by using a Doppler effect. The Doppler image may be a blood flow Doppler image showing flow of blood (also referred to as a color Doppler image), a tissue Doppler image showing a movement of tissue, or a spectral Doppler image showing a moving speed of an object as a waveform. According to an embodiment, the image processor 130 may extract Doppler components from ultrasound data, and may generate a Doppler image showing a movement of an object as colors or waveforms based on the extracted Doppler components.

The display 140 may display a generated ultrasound image and various pieces of information processed by the ultrasound diagnosis apparatus 100. The ultrasound diagnosis apparatus 100 may include two or more displays 140 according to the present exemplary embodiment. The display 140 may include a touch screen in combination with a touch panel.

The controller 120 may control the operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus.

The ultrasound diagnosis apparatus 100 may include the communication module 160 and may be connected to external apparatuses, for example, servers, medical apparatuses, and portable devices such as smart phones, tablet personal computers (PCs), wearable devices, etc., via the communication module 160.

The communication module 160 may include at least one element capable of communicating with the external apparatuses. For example, the communication module 160 may include at least one among a short-range communication module, a wired communication module, and a wireless communication module.

The communication module 160 may receive a control signal and data from an external apparatus and transmit the received control signal to the controller 120 so that the controller 120 may control the ultrasound diagnosis apparatus 100 in response to the received control signal.

The controller 120 may transmit a control signal to the external apparatus via the communication module 160 so that the external apparatus may be controlled in response to the control signal of the controller 120.

For example, the external apparatus connected to the ultrasound diagnosis apparatus 100 may process the data of the external apparatus in response to the control signal of the controller 120 received via the communication module 160.

A program for controlling the ultrasound diagnosis apparatus 100 may be installed in the external apparatus. The program may include command languages to perform part of operation of the controller 120 or the entire operation of the controller 120.

The program may be pre-installed in the external apparatus or may be installed by a user of the external apparatus by downloading the program from a server that provides applications. The server that provides applications may include a recording medium where the program is stored.

The storage 150 may store various data or programs for driving and controlling the ultrasound diagnosis apparatus 100, input and/or output ultrasound data, ultrasound images, applications, etc.

The input interface 170 may receive a user's input to control the ultrasound diagnosis apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

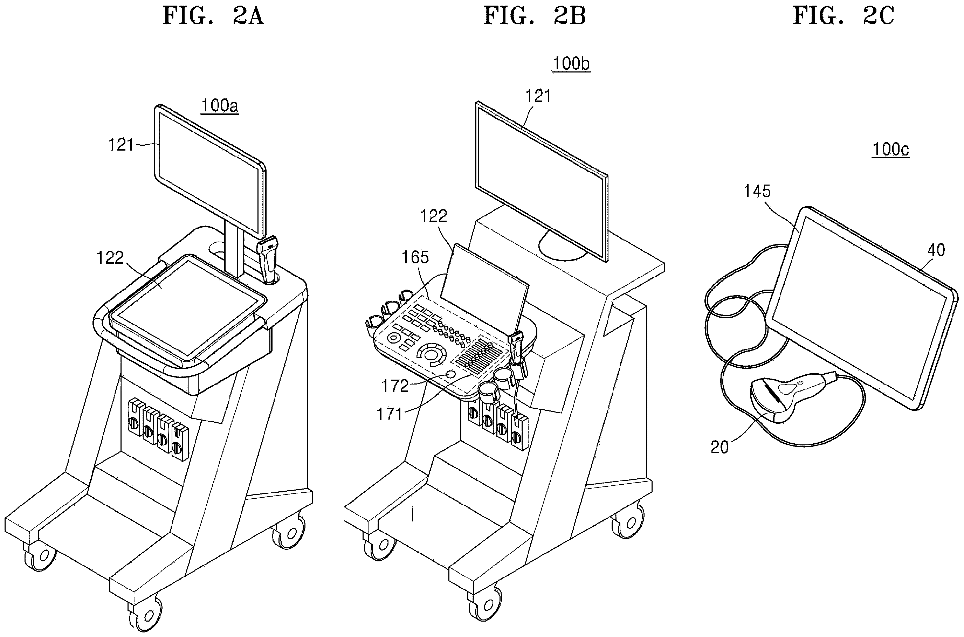

An example of the ultrasound diagnosis apparatus 100 according to the present exemplary embodiment is described below with reference to FIG. 2A, 2B, and 2C.

FIGS. 2A, 2B, and 2C are diagrams illustrating ultrasound diagnosis apparatus according to an exemplary embodiment.

Referring to FIGS. 2A and 2B, the ultrasound diagnosis apparatus 100a and 100b may include a main display 121 and a sub-display 122. At least one among the main display 121 and the sub-display 122 may include a touch screen. The main display 121 and the sub-display 122 may display ultrasound images and/or various information processed by the ultrasound diagnosis apparatus 100a and 100b. The main display 121 and the sub-display 122 may provide graphical user interfaces (GUI), thereby receiving user's inputs of data to control the ultrasound diagnosis apparatus 100. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound diagnosis apparatus 100b may control the display of the ultrasound image on the main display 121 by using the input control data.

Referring to FIG. 2B, the ultrasound diagnosis apparatus 100b may include a control panel 165. The control panel 165 may include buttons, trackballs, jog switches, or knobs, and may receive data to control the ultrasound diagnosis apparatus 100 from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound diagnosis apparatus 100b may keep displaying a frame image at that time point.

The buttons, trackballs, jog switches, and knobs included in the control panel 165 may be provided as a GUI to the main display 121 or the sub-display 122.

Referring to FIG. 2C, the ultrasound diagnosis apparatus 100c may include a portable device. An example of the portable ultrasound diagnosis apparatus 100c may include, for example, smart phones including probes and applications, laptop computers, personal digital assistants (PDAs), or tablet PCs, but an exemplary embodiment is not limited thereto.

The ultrasound diagnosis apparatus 100c may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound diagnosis apparatus 100c, and a GUI.

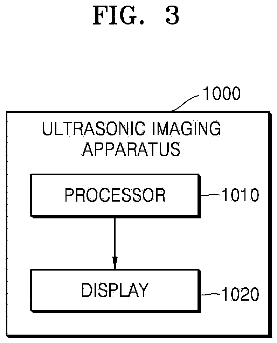

FIG. 3 is a block diagram of an ultrasonic imaging apparatus 1000 according to an embodiment.

The ultrasonic imaging apparatus 1000 may include a processor 1010 and a display 1020. According to an embodiment, the processor 1010 may correspond to at least one or a combination of the image processor 130 and the controller 120 described with reference to FIG. 1, and the display 1020 may correspond to the display 140 of FIG. 1.

FIG. 3 shows that the ultrasonic imaging apparatus 1000 includes only components related to the present embodiment. Thus, it will be understood by those of ordinary skill in the relevant art that the ultrasonic imaging apparatus 1000 may include other common components as well as the components shown in FIG. 3. For example, the ultrasonic imaging apparatus 1000 may include at least one of the probe 20, the ultrasound transceiver 110, the storage 150, the communication module 160, and the input interface 170 described with reference to FIG. 1.

The processor 1010 controls all operations of the ultrasonic imaging apparatus 1000 and processes data and signals. The processor 1010 may be composed of at least one hardware unit. According to an embodiment, the processor 1010 may include a separate hardware unit that serves as the image processor 130 and the controller 120. The processor 1010 may be implemented by one or more software modules that are generated by executing program code stored in a memory.

The processor 1010 may acquire a Doppler signal corresponding to a sample volume. In other words, the processor 1010 may acquire a Doppler signal corresponding to a sample volume set with respect to an object.

According to an embodiment, the ultrasonic imaging apparatus 1000 may include the ultrasound transceiver 110, and the ultrasound transceiver 110 may control the probe 20 to transmit, to a sample volume, an ultrasound signal which is generated according to a predetermined pulse repetition frequency (PRF) and receive an echo signal reflected from the sample volume. The processor 1010 may then extract Doppler components from the received echo signal to generate a Doppler signal. For example, the processor 1010 may perform quadrature demodulation on the received echo signal to acquire a complex baseband ultrasound signal as a Doppler signal. Thus, the processor 1010 may acquire a Doppler signal corresponding to the sample volume every period according to a PRF. Furthermore, the processor 1010 may acquire a Doppler signal corresponding to the sample volume repeatedly by an ensemble number.

According to another embodiment, the processor 1010 may acquire a Doppler signal corresponding to the sample volume from an external server or external device via the communication module 160 of the ultrasonic imaging apparatus 1000. According to another embodiment, the processor 1010 may acquire a prestored Doppler signal corresponding to the sample volume from the storage 150 of the ultrasonic imaging apparatus 1000.

The processor 1010 may divide an acquired Doppler signal into a plurality of sub-Doppler signals. According to an embodiment, the processor 1010 may divide a sample volume into a plurality of sections along a direction in which an ultrasound signal is transmitted and divide a Doppler signal into a plurality of sub-Doppler signals respectively corresponding to the plurality of sections. Furthermore, the processor 1010 may determine, based on a user input, the number and size of the plurality of sections set in the sample volume, and accordingly, determine the number and magnitude of the plurality of sub-Doppler signals.

Furthermore, the processor 1010 may divide each of a plurality of Doppler signals acquired every period according to a PRF into a plurality of sub-Doppler signals and repeatedly divide a Doppler signal into a plurality of sub-Doppler signals by an ensemble number.

The processor 1010 may generate a plurality of spectral Doppler signals by performing spectral analysis on each of a plurality of sub-Doppler signals. Spectral analysis refers to a process of estimating a Doppler signal in a time domain as a spectrum in a frequency domain, e. g., a process of converting a Doppler signal in the time domain into a spectral Doppler signal in the frequency domain. According to an embodiment, the spectral analysis may include performing weighting accumulation on a Doppler signal and performing fast Fourier transform (FFT) on the resulting signal. Thus, the processor 1010 may generate a plurality of spectral Doppler signals by performing weighting accumulation and FFT on the respective sub-Doppler signals.

The processor 1010 may generate a spectral Doppler signal corresponding to a sample volume from a plurality of spectral Doppler signals. According to an embodiment, the processor 1010 may generate a spectral Doppler signal corresponding to a sample volume by performing weighting accumulation on a plurality of spectral Doppler signals. According to another embodiment, the processor 1010 may determine at least one effective spectral Doppler signal from among a plurality of spectral Doppler signals and generate a spectral Doppler signal corresponding to a sample volume from the determined at least one spectral Doppler signal. For example, the processor 1010 may determine spectral Doppler signals representing an object from among a plurality of spectral Doppler signals and generate a spectral Doppler signal corresponding to a sample volume.

The display 1020 may display the generated spectral Doppler signal.

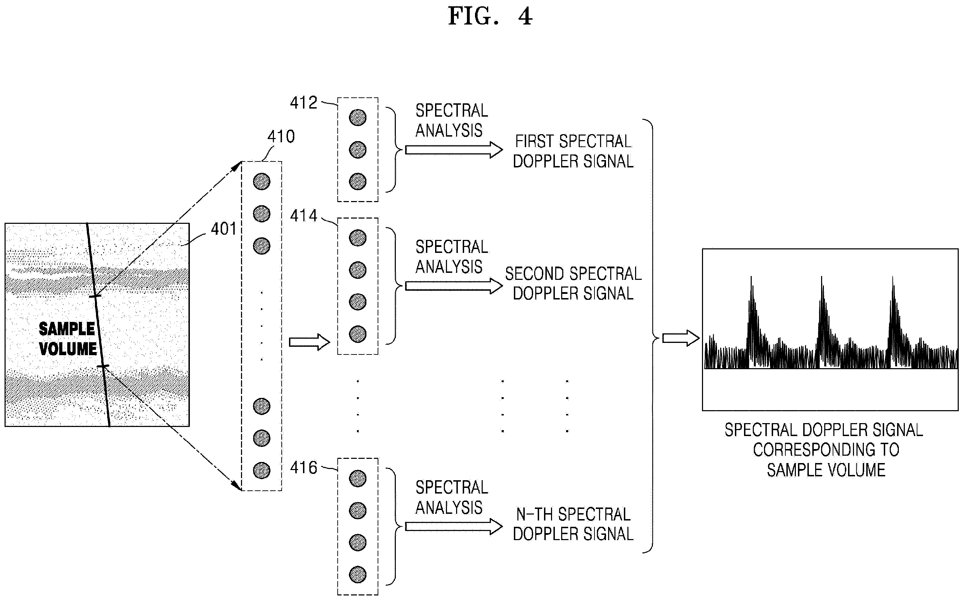

FIG. 4 is a diagram illustrating an embodiment in which the ultrasonic imaging apparatus 1000 generates a spectral Doppler signal corresponding to a sample volume.

According to an embodiment, the display 1020 may display a B-mode ultrasound image 401 showing an object. Subsequently, the processor 1010 may set a sample volume with respect to the object based on a user input. For example, the processor 1010 may set a sample volume with respect to a specific blood vessel.

The processor 1010 may acquire a Doppler signal 410 corresponding to the sample volume.

The processor 1010 may then divide the Doppler signal 410 into a plurality of sub-Doppler signals 412, 414, and 416. For example, the processor 1010 may divide the sample volume into first through N-th sections according to a direction in which an ultrasound signal is transmitted and divide the Doppler signal 410 into the plurality of sub-Doppler signals 412, 414, and 416 respectively corresponding to the first through N-th sections set in the sample volume.

Thereafter, the processor 1010 may generate a plurality of spectral Doppler signals by performing spectral analysis on each of the sub-Doppler signals 412, 414, and 416. In detail, the processor 1010 may generate first through N-th spectral Doppler signals by performing spectral analysis such as weighting accumulation and FFT on the sub-Doppler signals 412, 414, and 416.

The processor 1010 may then generate, from the plurality of spectral Doppler signals, a spectral Doppler signal corresponding to the sample volume. According to an embodiment, the processor 1010 may generate a spectral Doppler signal corresponding to the sample volume by performing weighting accumulation on the plurality of spectral Doppler signals.

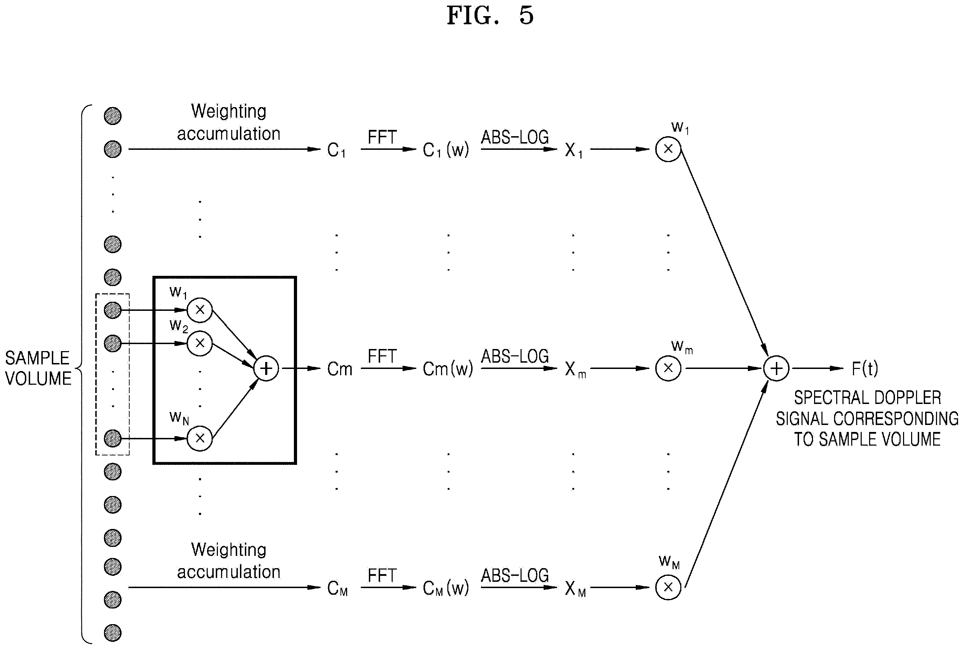

FIG. 5 is a diagram illustrating a detailed embodiment in which an ultrasonic imaging apparatus generates a spectral Doppler signal corresponding to a sample volume.

The processor 1010 may divide a Doppler signal corresponding to a sample volume into a plurality of sub-Doppler signals and perform spectral analysis on each of the plurality of sub-Doppler signals.

First, the processor 1010 may perform weighting accumulation on each of the plurality of sub-Doppler signals. In detail, the processor 1010 may perform weighting accumulation on an m-th sub-Doppler signal from among a number of M sub-Doppler signals by using Equation (1) below:

$$c_m(t,1) = \sum_{n=0}^{N} w(n) \cdot S_m(t + \Delta t, n)$$

$$\ldots(1)$$

In Equation (1), $S_m(t+\Delta t, n)$ denotes a sub-Doppl $c_m(t,1)$ (510) having a time interval of $\Delta t = 1/PRF$ and N pieces of sample data in a depth direction, w(n) denotes a window function for weighting accumulation, and $c_m(t, 1)$ denotes a m-th sub-Doppler signal that has undergone the weighting accumulation. According to an embodiment, the window function w(n) for weighting accumulation may be a hanning window function as shown in Equation (2). According to another embodiment, the window function w(n) may be a trigometric window function, a Daniel window function, or a hamming window function.

$$w(n) = \begin{cases} \frac{1}{2}\left[1 - \cos\left(\frac{2\pi n}{N}\right)\right], & 0 \le n \le N \\ 0 \end{cases}$$

$$\ldots(2)$$

Subsequently, the processor 1010 may perform FFT on the Doppler signal $c_m$ that has undergone the weighting accumulation by using Equation (3) below:

$$c_m(\omega) = \sum_{p=0}^{p-1} c_m e^{-jp\omega T}$$

...(3)

In Equation (3), the Doppler signal $c_m$ that has undergone the weighting accumulation may denote P data sampled with a period T = $\triangle$t in the time domain. Thus, the processor 1010 may perform weighting accumulation and FFT on each of the M sub-Doppler signals by using Equations (1) through (3).

Then, the processor 1010 may generate a spectral Doppler signal $X_m(t)$ by performing an absolute value (ABS) operation and log transformation on a Doppler signal $c_m(\omega)$ that has undergone Fourier transformation by using Equation (4) below:

$$X_m(t) = \log_{10}\left(\left|C_m(\omega)\right|\right)$$

... (4)

The processor 1010 may then generate a spectral Doppler signal F(t) corresponding to a sample volume by performing weighting accumulation on a plurality of spectral Doppler signals $X_1$ through $X_M$ by using Equation (5) below:

$$F(t) = \sum_{m=0}^{M} w_m \cdot X_m(t)$$

... (5)

According to an embodiment, in Equation (5), a window function $w_m$ for weighting accumulation may be a hanning window function, a trigometric window function, a Daniel window function, a hamming window function, or the like.

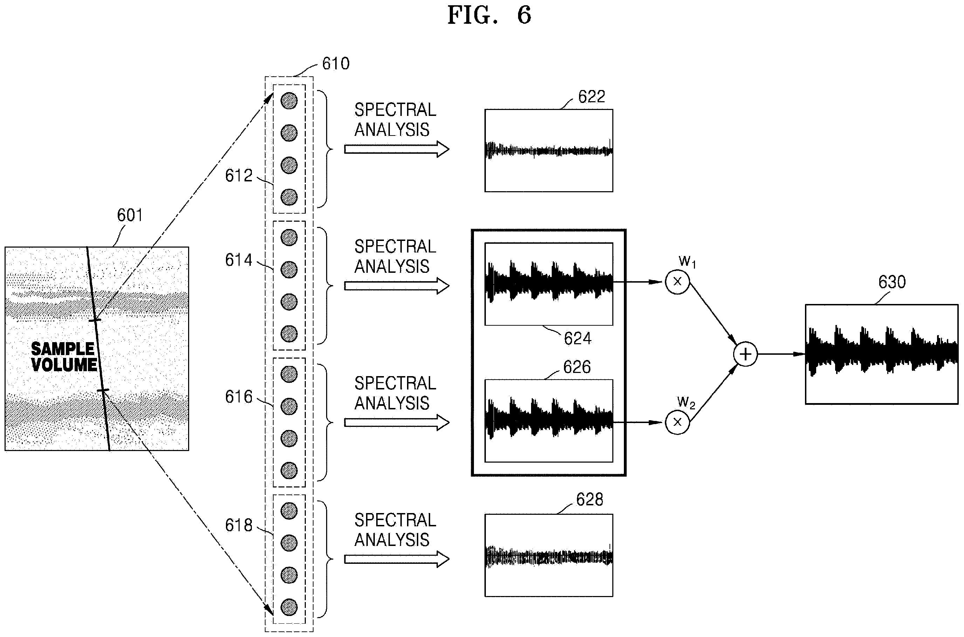

FIG. 6 is a diagram illustrating an embodiment in which an ultrasonic imaging apparatus generates a spectral Doppler signal corresponding to a sample volume.

The processor 1010 may acquire a Doppler signal 610 corresponding to a sample volume set in an ultrasound image 601. The processor 1010 may then divide the Doppler signal 610 into sub-Doppler signals 612, 614, 616, and 618. Subsequently, the processor 1010 may generate spectral Doppler signals 622, 624, 626, and 628 by performing spectral analysis on each of the sub-Doppler signals 612, 614, 616, and 618. Although FIG. 6 shows four (4) sub-Doppler signals and four spectral Doppler signals, the number of sub-Doppler signals and the number of spectral Doppler signals are not limited thereto.

Then, the processor 1010 may then determine the spectral Doppler signals 624 and 626 to be effective from among the spectral Doppler signals 622, 624, 626, and 628. According to an embodiment, the processor 1010 may determine the spectral Doppler signals 624 and 626 containing relatively little artifact among the spectral Doppler signals 622, 624, 626, and 628 as effective spectral Doppler signals. According to another embodiment, when the sample volume is set with respect to a specific blood vessel, the processor 1010 may determine the spectral Doppler signals 624 and 626 representing the specific blood vessel from among the spectral Doppler signals 622, 624, 626, and 628 as effective spectral Doppler signals. Even though the sample volume is set with respect to a specific blood vessel, a section of the sample volume may not include the specific blood vessel when the blood vessel is small or a patient moves.

Next, the processor 1010 may generate a spectral Doppler signal 630 corresponding to the sample volume by using the effective spectral Doppler signals 624 and 626. According to an embodiment, the processor 1010 may generate the spectral Doppler signal 630 by performing weighting accumulation on the spectral Doppler signals 624 and 626 determined to be effective.

Thus, the ultrasonic imaging apparatus 1000 may divide a Doppler signal corresponding to a sample volume into a plurality of sub-Doppler signals and generate a spectral Doppler signal corresponding to the sample volume from a plurality of spectral Doppler signals respectively corresponding to the plurality of sub-Doppler signals. Thus, the special Doppler signal corresponding to the sample volume may be displayed as a high quality image. In other words, by displaying a spectral Doppler signal corresponding to the sample volume by partially performing weighting accumulation and Fourier transformation on the Doppler signal, the ultrasonic imaging apparatus 1000 is capable of displaying an image from which speckle noise is removed and with a clear boundary of a Doppler spectrum.

Furthermore, the ultrasonic imaging apparatus 1000 may generate a spectral Doppler signal corresponding to the sample volume by determining an effective spectral Doppler signal from among a plurality of spectral Doppler signals respectively corresponding to sections of the sample volume. Thus, the ultrasonic imaging apparatus 1000 may generate and display a more accurate spectral Doppler signal corresponding to the sample volume.

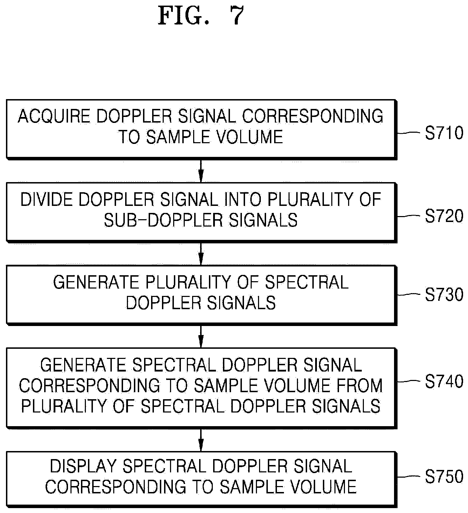

FIG. 7 is a flowchart of a method whereby an ultrasonic imaging apparatus displays an ultrasound image, according to an embodiment.

The method illustrated in FIG. 7 may be performed by each of the components of the ultrasonic imaging apparatus 1000 of FIG. 3, and repeated descriptions with respect to FIG. 3 will be omitted here.

In operation S710, the ultrasonic imaging apparatus 1000 may acquire a Doppler signal corresponding to a sample volume.

According to an embodiment, the ultrasonic imaging apparatus 1000 may control a probe to transmit, to a sample volume, an ultrasound signal which is generated according to a predetermined pulse repetition frequency (PRF) and receive an echo signal reflected from the sample volume. The ultrasonic imaging apparatus 1000 may then extract Doppler components with respect to the received echo signal to generate a Doppler signal.

According to another embodiment, the ultrasonic imaging apparatus 1000 may acquire a Doppler signal corresponding to a sample volume from an external server or an external device via a communication module. According to another embodiment, the ultrasonic imaging apparatus 1000 may acquire a Doppler signal corresponding to the sample volume prestored in a storage.

In operation S720, the ultrasonic imaging apparatus 1000 may divide the Doppler signal into a plurality of sub-Doppler signals. According to an embodiment, the ultrasonic imaging apparatus 1000 may divide the sample volume into a plurality of sections along a direction in which an ultrasound signal is transmitted and divide the Doppler signal into a plurality of sub-Doppler signals respectively corresponding to the plurality of sections. Furthermore, the ultrasonic imaging apparatus 1000 may determine, based on a user input, the number and size of the plurality of sections set in the sample volume, and accordingly, determine the number and magnitude of the plurality of sub-Doppler signals.

In operation S730, the ultrasonic imaging apparatus 1000 may generate a plurality of spectral Doppler signals by performing spectral analysis on each of the sub-Doppler signals. The ultrasonic imaging apparatus 1000 may generate a plurality of spectral Doppler signals by performing weighting accumulation and FFT with respect to the respective sub-Doppler signals.

In operation S740, the ultrasonic imaging apparatus 1000 may generate, from the plurality of spectral Doppler signals, a spectral Doppler signal corresponding to the sample volume.

According to an embodiment, the ultrasonic imaging apparatus 1000 may generate a spectral Doppler signal corresponding to the sample volume by performing weighting accumulation on the plurality of spectral Doppler signals. According to another embodiment, the ultrasonic imaging apparatus 1000 may determine at least one effective spectral Doppler signal from among the plurality of spectral Doppler signals and generate a spectral Doppler signal corresponding to the sample volume based on the determined at least one spectral Doppler signal.

In operation S750, the ultrasonic imaging apparatus 1000 may display the spectral Doppler signal corresponding to the sample volume.

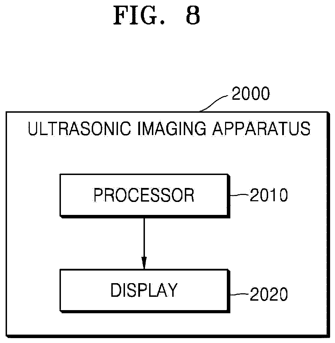

FIG. 8 is a block diagram of an ultrasonic imaging apparatus 2000 according to another embodiment.

The ultrasonic imaging apparatus 2000 may include a processor 2010 and a display 2020. According to an embodiment, the processor 2010 may correspond to at least one or a combination of the image processor 130 and the controller 120 described with reference to FIG. 1, and the display 2020 may correspond to the display 140 of FIG. 1.

FIG. 8 shows that the ultrasonic imaging apparatus 2000 includes only components related to the present embodiment. Thus, it will be understood by those of ordinary skill in the relevant art that the ultrasonic imaging apparatus 2000 may include other common components as well as the components shown in FIG. 8. For example, the ultrasonic imaging apparatus 2000 may include at least one of the probe 20, the ultrasound transceiver 110, the storage 150, the communication module 160, and the input interface 170 described with reference to FIG. 1.

Furthermore, because the processor 2010 and the display 2020 may respectively correspond to the processor 1010 and the display 1020 described with reference to FIG. 3, repeated descriptions with respect to FIG. 3 will be omitted here.

The processor 2010 may acquire a Doppler signal corresponding to a sample volume.

The processor 2010 may divide an acquired Doppler signal into a plurality of sub-Doppler signals respectively corresponding to a plurality of sections set in the sample volume. In detail, the processor 2010 may segment the sample volume into a plurality of sections along a direction in which an ultrasound signal is transmitted or an axial direction of the sample volume and divide the Doppler signal into a plurality of sub-Doppler signals respectively corresponding to the plurality of sections. Furthermore, the processor 2010 may determine, based on a user input, the number and size of the plurality of sections set in the sample volume, and accordingly, determine the number and magnitude of the plurality of sub-Doppler signals.

The processor 2010 may generate a plurality of spectral Doppler signals by performing spectral analysis on each of the plurality of sub-Doppler signals. According to an embodiment, the processor 2010 may generate a plurality of spectral Doppler signals by performing weighting accumulation and FFT on the respective sub-Doppler signals.

According to an embodiment, when the sample volume is set with respect to a blood vessel, the processor 2010 may provide blood flow characteristics in a plurality of sections set in the sample volume by using a plurality of spectral Doppler signals respectively corresponding to the plurality of sections. For example, for each of a plurality of sections, the processor 2010 may provide a user with changes in frequency and velocity of blood flow with respect to time, and may also calculate and provide to the user a peak velocity, a mean velocity, a peak gradient, an acceleration time, a deceleration time, a peak systolic velocity (PSV), an end diastolic velocity, a resistive index, a pulsatility index, etc.

The display 2020 may display the generated spectral Doppler signals.

Furthermore, according to an embodiment, the processor 2010 may generate a plurality of spectral lines respectively corresponding to the plurality of sections of the sample volume by respectively performing line tracing on the plurality of spectral Doppler signals, and the display 2020 may display the plurality of spectral lines. Line tracing is a technique for detecting a mean or peak velocity of a spectrum represented by a spectral Doppler signal and tracing a level of the detected mean or peak velocity.

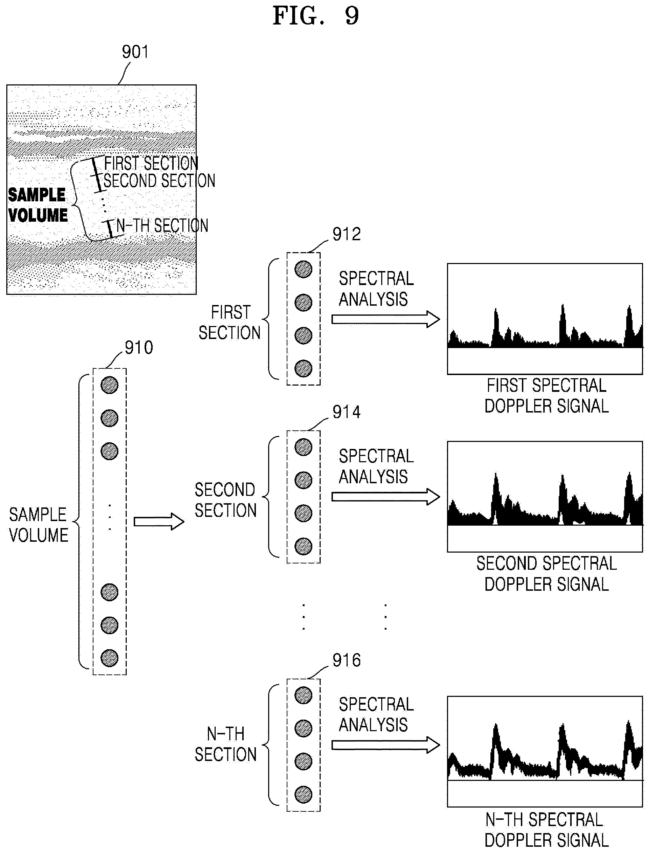

FIG. 9 illustrates an embodiment in which the ultrasonic imaging apparatus 2000 displays a plurality of spectral Doppler signals.

According to an embodiment, the processor 2010 may acquire a Doppler signal 910 corresponding to a sample volume set in a B-mode ultrasound image 901. Furthermore, the processor 2010 may set a plurality of sections in the sample volume. In detail, the processor 2010 may set a plurality of sections along an axial direction on the sample volume. According to an embodiment, as shown in FIG. 9, the processor 2010 may divide the sample volume into first through N-th sections. For example, when the sample volume is set with respect to a blood vessel, the first section may be a section close to a blood vessel wall, and the N-th section may be a section close to an opposite blood vessel wall.

The processor 2010 may then divide the Doppler signal 910 into a plurality of sub-Doppler signals 912, 914, and 916 respectively corresponding to the plurality of sections set in the sample volume. In detail, the processor 2010 may divide the Doppler signal 910 into the sub-Doppler signals 912, 914, and 916 respectively corresponding to the first, second, and N-th sections of the sample volume.

Subsequently, the processor 2010 may generate a plurality of spectral Doppler signals respectively corresponding to the plurality of sections by performing spectral analysis on each of the sub-Doppler signals 912, 914, and 916. According to an embodiment, the processor 2010 may generate a plurality of spectral Doppler signals by performing weighting accumulation and FFT on the respective sub-Doppler signals 912, 914, and 916. For example, the processor 2010 may generate a plurality of spectral Doppler signals by applying Equations (1) through (4) of FIG. 5 to the respective sub-Doppler signals 912, 914, and 916. Thus, the processor 2010 may generate first, second, and N-th spectral Doppler signals respectively corresponding to the first, second, and N-th sections of the sample volume.

Then, the display 2020 may display the generated spectral Doppler signals.

Thus, the ultrasonic imaging apparatus 2000 may simultaneously display spectral Doppler signals respectively corresponding to a plurality of sections set in a sample volume, thereby providing information about the plurality of sections of the sample volume to the user. For example, when the sample volume is set with respect to a blood vessel, the ultrasonic imaging apparatus 2000 may simultaneously provide a user with various pieces of blood flow information regarding the plurality of sections in the blood vessel. Furthermore, the ultrasonic imaging apparatus may display spectral Doppler signals respectively corresponding to the sections of the sample volume based on a Doppler signal acquired every period according to a PRF, thereby providing information regarding each section of the sample volume during the same time.

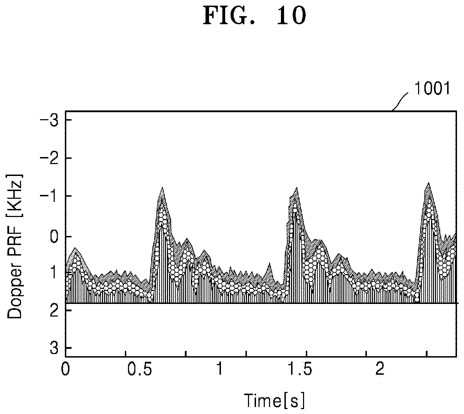

FIG. 10 illustrates an embodiment in which the ultrasonic imaging apparatus 2000 displays a plurality of spectral Doppler signals as a single image.

According to an embodiment, the processor 2010 may overlap a plurality of spectral Doppler signals respectively corresponding to a plurality of sections set in a sample volume with one another. Then, the display 2020 may display the overlapped spectral Doppler signals as a single image.

Furthermore, according to an embodiment, the processor 2010 may generate a Doppler spectrum 1001 in which the overlapped spectral Doppler signals are respectively mapped in different colors. Then, the display 2020 may display the Doppler spectrum 1001. In other words, the display 2020 may respectively display spectral Doppler signals corresponding to first, second, and N-th sections of the sample volume in first, second, and N-th colors.

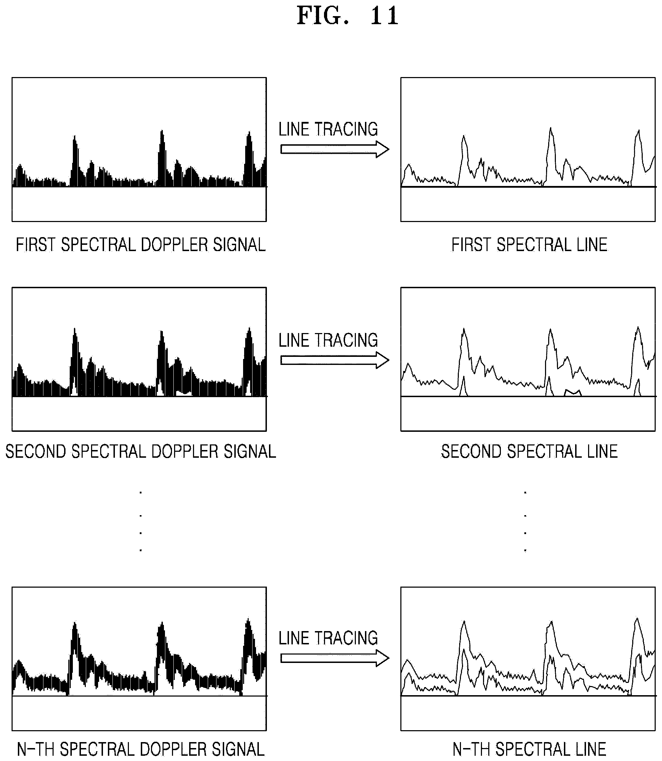

FIG. 11 illustrates an embodiment in which the ultrasonic imaging apparatus 2000 displays a plurality of spectral Doppler signals.

The processor 2010 may generate a plurality of spectral lines respectively corresponding to a plurality of sections set in a sample volume by respectively performing line tracing on a plurality of spectral Doppler signals. In detail, the processor 2010 may generate first, second, and N-th spectral lines respectively corresponding to first, second, and N-th spectral Doppler signals.

Thus, the processor 2010 may generate and control the display 2020 to display not only a plurality of spectral Doppler signals representing the entire spectrum as shown in FIG. 9 but also spectral lines of the plurality of spectral Doppler signals as shown in FIG. 11.

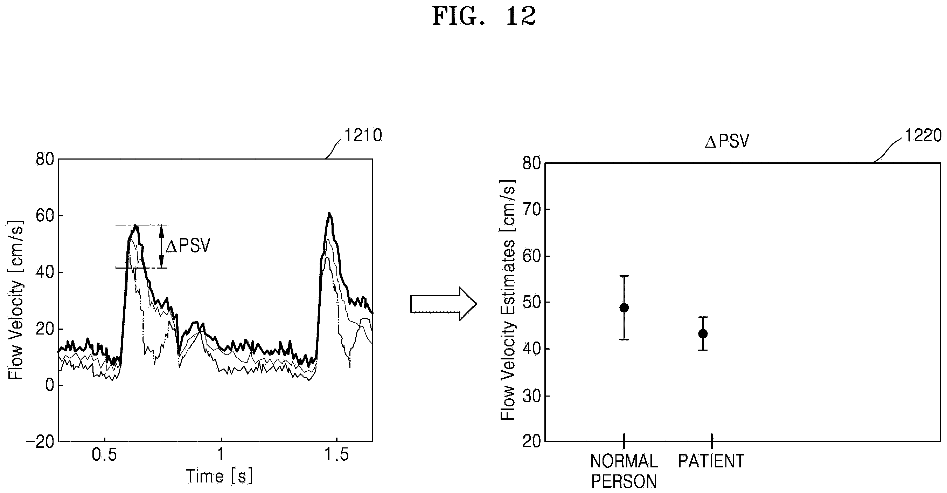

FIG. 12 illustrates an embodiment in which the ultrasonic imaging apparatus 2000 provides information about vascular wall stiffness.

According to an embodiment, the processor 2010 may generate a single image by superimposing on one another spectral lines of a plurality of spectral Doppler signals respectively corresponding to a plurality of sections set in a sample volume.

Furthermore, the processor 2010 may detect a PSV in each of the plurality of sections of the sample volume based on the plurality of spectral Doppler signals or spectral lines. Furthermore, the processor 2010 may detect a difference between PSVs in the respective sections of the sample volume. For example, when the sample volume is set with respect to an artery, the processor 2010 may generate a spectral Doppler signal or a spectral line for each section of the artery. In other words, the processor 2010 may respectively generate spectral Doppler signals or spectral lines in a wall and a central section of the artery. Thus, the processor 2010 may detect a PSV in each section of the artery based on a spectral Doppler signal or a spectral line generated for each section of the artery and detect a difference between PSVs in the respective sections of the artery.

Referring to FIG. 12, when the sample volume is set with respect to a blood vessel, the processor 2010 may respectively generate spectra lines for sections of the blood vessel, and the display 2020 may display an image 1210 showing the generated spectra lines. Furthermore, the processor 2010 may detect a PSV in each of the sections of the blood vessel based on a spectral line corresponding to each section of the blood vessel. In addition, the processor 2010 may detect a difference △PSV between maximum and minimum values selected from among PSVs detected in the respective sections of the blood vessel. For example, the difference △PSV may be a difference between PSVs in a section near a blood vessel wall and a central section of the blood vessel.

The processor 2010 may generate information about vascular wall stiffness based on a difference △PSV. In the case of a blood vessel, a difference between blood flow velocities in each section of the blood vessel may vary according to vascular wall stiffness. For example, when the vascular wall stiffness increases, a difference between blood flow velocities in a central section and a wall of the blood vessel may decrease. When the vascular wall stiffness decreases, a difference between blood flow velocities in the central section and wall of the blood vessel may increase. Thus, the processor 2010 may detect a difference APSV for a patient's blood vessel and assess vascular wall stiffness in the patient based on the difference △PSV.

According to an embodiment, the processor 2010 may generate a graph 1220 as information about vascular wall stiffness. In detail, the processor 2010 may set a sample volume in a patient's blood vessel, generate a plurality of spectral Doppler signals respectively corresponding to a plurality of sections of the blood vessel, and detect a difference △PSV for the blood vessel from the plurality of spectral Doppler signals. Furthermore, the processor 2010 may generate the graph 1220 for comparing detected difference △PSV for the patient with a prestored difference △PSV for a normal person. Thus, by displaying the graph 1220, the display 2020 may provide the patient with information representing comparison between vascular wall stiffness in the patient and vascular wall stiffness in the normal person.

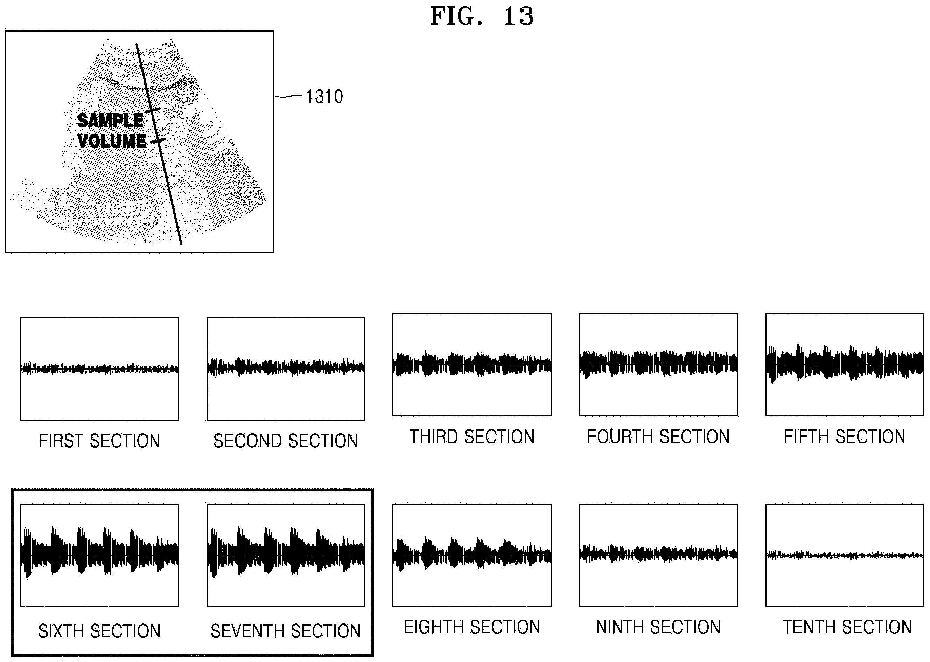

FIG. 13 illustrates an embodiment in which the ultrasonic imaging apparatus 2000 displays a plurality of spectral Doppler signals.

The processor 2010 may set, based on a user input, a sample volume with respect to a blood vessel in a B-mode ultrasound image 1310. For example, the processor 21010 may set a sample volume with respect to a microscopic blood vessel in a kidney.

Next, the processor 2010 may acquire a Doppler signal corresponding to the sample volume and divide the acquired Doppler signal into first through tenth sub-Doppler signals respectively corresponding to first through tenth sections of the sample volume. The processor 2010 may then generate spectral Doppler signals respectively corresponding to the first through tenth sections of the sample volume by performing spectral analysis on each of the first through tenth sub-Doppler signals. As shown in FIG. 13, the display 2020 may display the spectral Doppler signals respectively corresponding to the first through tenth sections of the sample volume.

According to an embodiment, the ultrasonic imaging apparatus 2000 may include an input interface 170 via which a user may select at least one of spectral Doppler signals displayed on the display 2020. After selecting the at least one spectral Doppler signal, the display 2020 may display only the selected at least one spectral Doppler signal. For example, when the sample volume is set in a region including a microscopic blood vessel, it may be difficult to observe a spectral Doppler signal corresponding to the microscopic blood vessel due to movement such as human body's respiration. Thus, the ultrasonic imaging apparatus 2000 may provide the user with spectral Doppler signals respectively corresponding to the first through tenth sections, and the user may select, from among the provided spectral Doppler signals, spectral Doppler signals that respectively correspond to the sixth and seventh sections and best represent the microscopic blood vessel. Furthermore, according to an embodiment, the processor 2010 may perform weighting accumulation on the spectral Doppler signals, respectively corresponding to the sixth and seventh sections and being selected by the user, and display 2020 may display the spectral Doppler signals that have undergone the weighting accumulation.

According to another embodiment, the processor 2010 may select at least one effective spectral Doppler signal from among the spectral Doppler signals respectively corresponding to the first through tenth sections. Then, the display 2020 may display the selected at least one effective spectral Doppler signal. For example, when the sample volume is set in a region including a blood vessel, the processor 2010 may select a spectral Doppler signal that best represents characteristics of the blood vessel from among the spectral Doppler signals respectively corresponding to the first through tenth sections. During this process, the processor 2010 may exclude, from the spectral Doppler signals, a spectral Doppler signal representing a spectrum of ultrasound signals reflected from a tissue or containing much artifact.

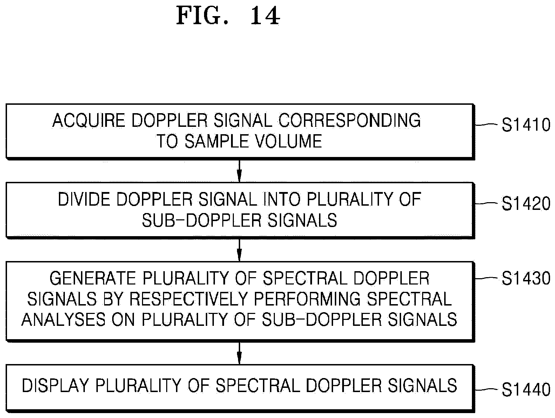

FIG. 14 illustrates a method whereby an ultrasonic imaging apparatus displays an ultrasound image, according to an embodiment.

The method illustrated in FIG. 14 may be performed by each of the components of the ultrasonic imaging apparatus 2000 of FIG. 8, and repeated descriptions with respect to FIG. 8 will be omitted here.

In operation S1410, the ultrasonic imaging apparatus 2000 may acquire a Doppler signal corresponding to a sample volume.

In operation S1420, the ultrasonic imaging apparatus 2000 may divide the Doppler signal into a plurality of sub-Doppler signals respectively corresponding to a plurality of sections set in the sample volume.

The ultrasonic imaging apparatus 2000 may divide the sample volume into a plurality of sections along a direction in which an ultrasound signal is transmitted or an axial direction of the sample volume and then divide the Doppler signal into a plurality of sub-Doppler signals respectively corresponding to the plurality of sections. Furthermore, the ultrasonic imaging apparatus 2000 may determine, based on a user input, the number and size of the plurality of sections set in the sample volume, and accordingly, determine the number and magnitude of the plurality of sub-Doppler signals.

In operation S1430, the ultrasonic imaging apparatus 2000 may generate a plurality of spectral Doppler signals respectively corresponding to the plurality of sections by performing spectral analysis on each of the sub-Doppler signals. According to an embodiment, the ultrasonic imaging apparatus 2000 may generate a plurality of spectral Doppler signals by performing weighting accumulation and FFT on each of a plurality of sub-Doppler signals.

In operation S1440, the ultrasonic imaging apparatus 2000 may display the plurality of spectral Doppler signals.

Furthermore, the ultrasonic imaging apparatus 2000 may generate and display a plurality of spectral lines respectively corresponding to the plurality of sections of the sample volume by respectively performing line tracing on the plurality of spectral Doppler signals.

Furthermore, when the sample volume is set with respect to a blood vessel, the ultrasonic imaging apparatus 2000 may generate and display information about vascular wall stiffness based on the plurality of spectral Doppler signals.

In addition, when the sample volume is set with respect to an object, the ultrasonic imaging apparatus 2000 may select at least one spectral Doppler signal representing the object from among the plurality of spectral Doppler signals and display the selected at least one spectral Doppler signal.

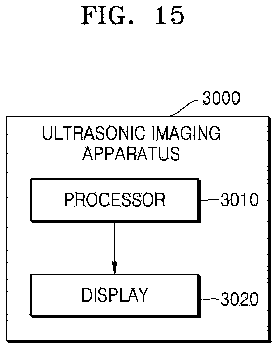

FIG. 15 is a block diagram of an ultrasonic imaging apparatus 3000 according to another embodiment.

The ultrasonic imaging apparatus 3000 may include a processor 3010 and a display 3020. According to an embodiment, the processor 3010 may correspond to at least one or a combination of the image processor 130 and the controller 120 described with reference to FIG. 1, and the display 3020 may correspond to the display 140 of FIG. 1.

FIG. 15 shows that the ultrasonic imaging apparatus 3000 includes only components related to the present embodiment. Thus, it will be understood by those of ordinary skill in the relevant art that the ultrasonic imaging apparatus 3000 may include other common components as well as the components shown in FIG. 15. For example, the ultrasonic imaging apparatus 3000 may include at least one of the probe 20, the ultrasound transceiver 110, the storage 150, the communication module 160, and the input interface 170 described with reference to FIG. 1.

Furthermore, because the processor 3010 may correspond to the processors 1010 and 2010 respectively described with reference to FIGS. 3 and 8, and the display 3020 may correspond to the displays 1020 and 2020 respectively described with reference to FIGS. 3 and 8, descriptions that are already provided above with respect to FIGS. 3 and 8 will be omitted here.

The processor 3010 may acquire a Doppler signal corresponding to a sample volume that is set with respect to an object. The sample volume may be set with respect to a region including the object. For example, the sample volume may be set with respect to a region including a specific blood vessel. Furthermore, the processor 3010 may set a plurality of sections in the sample volume. In detail, the processor 3010 may set a plurality of sections in the sample volume along an axial direction on the sample volume.

The processor 3010 may generate a plurality of spectral Doppler signals respectively corresponding to the plurality of sections by performing spectral analysis on each of the Doppler signal in the plurality of sections. According to an embodiment, the processor 3010 may divide the Doppler signal into a plurality of sub-Doppler signals respectively corresponding to the plurality of sections set in the sample volume and perform a plurality of spectral analyses on the sub-Doppler signals to generate a plurality of spectral Doppler signals respectively corresponding to the plurality of sections. According to an embodiment, the processor 3010 may generate a plurality of spectral Doppler signals by performing weighting accumulation and FFT on the respective sub-Doppler signals. For example, the processor 3010 may generate a plurality of spectral Doppler signals by applying Equations (1) through (4) of FIG. 5 to the respective sub-Doppler signals.

The processor 3010 may model an artifact signal in a spectral Doppler signal corresponding to an object by using a plurality of spectral Doppler signals.

First, the processor 3010 determines a spectral Doppler signal corresponding to an object from among a plurality of spectral Doppler signals. In detail, according to the invention, the processor 3010 determines a spectral Doppler signal corresponding to a section including an object from among a plurality of sections in a sample volume as a spectral Doppler signal corresponding to the object. According to another embodiment, the processor 3010 may generate a spectral Doppler signal corresponding to an object by performing weighting summation on spectral Doppler signals corresponding to two or more sections including the object from among a plurality of sections of a sample volume.

Next, the processor 3010 determines an artifact signal in the spectral Doppler signal corresponding to the object. In detail, the spectral Doppler signal corresponding to the object may include not only a signal representing the object but also an ultrasound reflected signal from another object. In other words, the spectral Doppler signal corresponding to the object may be a signal in which a signal representing the object is superimposed with an ultrasound reflected signal from another object. For example, when an object is a specific blood vessel, a spectral Doppler signal corresponding to the object may be a signal where a signal representing the specific blood vessel is superimposed with an ultrasound reflected signal from another blood vessel or a particular tissue. Thus, the processor 3010 determines as an artifact signal an ultrasound reflected signal from another object, which is contained in the spectral Doppler signal corresponding to the object.

The processor 3010 then models the artifact signal. According to the invention, the processor 3010 models the artifact signal by using a spectral Doppler signal corresponding to a section including another object from among the plurality of spectral Doppler signals. For example, when a spectral Doppler signal corresponding to one section including another object is included among the plurality of spectral Doppler signals, the processor 3010 may determine the spectral Doppler signal corresponding to the section including the other object as an artifact signal. As another example, when spectral Doppler signals respectively corresponding to two or more sections including another object are included among the plurality of spectral Doppler signals, the processor 3010 may generate an artifact signal by performing weighting summation on the spectral Doppler signals corresponding to the sections including the other object.

Based on the spectral Doppler signal corresponding to the object and the artifact signal, the processor 3010 may generate a spectral Doppler signal corresponding to the object and from which an artifact is removed. According to the invention, the processor 3010 generates a spectral Doppler signal corresponding to the object and from which an artifact is removed by performing subtraction between the spectral Doppler signal corresponding to the object and the artifact signal. In other words, the processor 3010 may remove a signal representing another object from the spectral Doppler signal corresponding to the object by subtracting the artifact signal from the spectral Doppler signal corresponding to the object.

The display 3020 displays the spectral Doppler signal corresponding to the object and from which the artifact is removed.

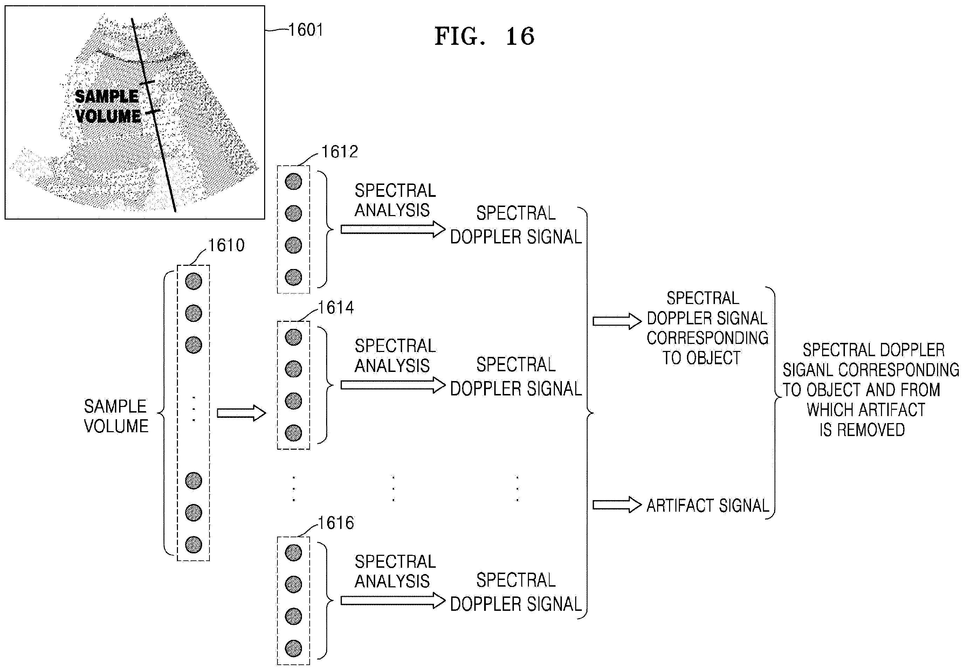

FIG. 16 illustrates an embodiment in which the ultrasonic imaging apparatus 3000 generates a spectral Doppler signal corresponding to an object.

The processor 3010 may acquire a Doppler signal 1610 corresponding to a sample volume set in a B-mode ultrasound image 1601. Furthermore, the processor 3010 may set a plurality of sections in the sample volume.

The processor 3010 may then divide the Doppler signal 1610 into a plurality of sub-Doppler signals 1612, 1614, and 1616 respectively corresponding to the plurality of sections set in the sample volume.

The processor 3010 may generate a plurality of spectral Doppler signals respectively corresponding to the plurality of sections by performing spectral analysis on each of the sub-Doppler signals 1612, 1614, and 1616.

The processor 3010 may model an artifact signal in a spectral Doppler signal corresponding to an object by using the plurality of spectral Doppler signals.

First, the processor 3010 may determine a spectral Doppler signal corresponding to a section including an object from among the plurality of sections of the sample volume as a spectral Doppler signal corresponding to the object.

Next, the processor 3010 may determine an artifact signal in the spectral Doppler signal corresponding to the object. The processor 3010 may determine as an artifact signal an ultrasound reflected signal from another object, which is contained in the spectral Doppler signal corresponding to the object.

The processor 3010 may then determine a spectral Doppler signal corresponding to a section including another object as an artifact signal.

The processor 3010 may generate, based on the spectral Doppler signal corresponding to the object and the artifact signal, a spectral Doppler signal corresponding to the object and from which an artifact is removed.

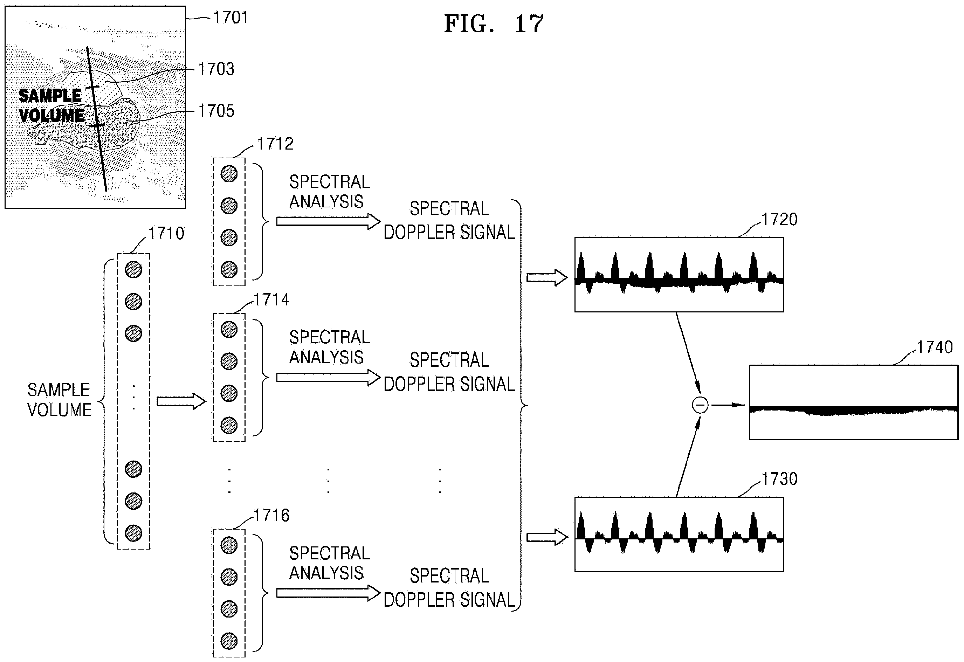

FIG. 17 illustrates a detailed embodiment in which the ultrasonic imaging apparatus 3000 generates a spectral Doppler signal corresponding to an object.

The processor 3010 may acquire a Doppler signal 1710 corresponding to a sample volume set in an ultrasound image 1701. The sample volume may be set to include a venous region 1703 and an arterial region 1705.

The processor 3010 may divide the Doppler signal 1710 into a plurality of sub-Doppler signals 1712, 1714, and 1716 respectively corresponding to a plurality of sections set in the sample volume. The processor 3010 may then generate a plurality of spectral Doppler signals respectively corresponding to the plurality of sections by performing spectral analysis on each of the sub-Doppler signals 1712, 1714, and 1716.

The processor 3010 may determine a spectral Doppler signal corresponding to a section including a vein from among the plurality of sections of the sample volume as a spectral Doppler signal 1720 corresponding to an object. The spectral Doppler signal 1720 corresponding to the object may be a signal in which a spectral Doppler signal representing the vein is superimposed with an ultrasound reflected signal from an artery. Thus, the processor 3010 may determine the ultrasound reflected signal from the artery as an artifact signal in the spectral Doppler signal 1720 corresponding to the object.

The processor 3010 may determine as an artifact signal 1730 a spectral Doppler signal corresponding to a section including the artery from among the plurality of spectral Doppler signals.

The processor 3010 may generate, based on the spectral Doppler signal 1720 corresponding to the object and the artifact signal 1730, a spectral Doppler signal 1740 corresponding to the object and from which an artifact is removed. In other words, the processor 3010 may generate the spectral Doppler signal 1740 as a signal obtained by removing the ultrasound reflected signal from the artery from the spectral Doppler signal 1720.

According to another embodiment, unlike in FIG. 17, the processor 3010 may determine a spectral Doppler signal corresponding to a section including an artery from among the plurality of sections of the sample volume as a spectral Doppler signal corresponding to an object, and a spectral Doppler signal corresponding to a section including a vein from among the plurality of spectral Doppler signals as an artifact signal. In other words, because the spectral Doppler signal corresponding to the object may include not only a spectral Doppler signal representing the artery but also an ultrasound reflected signal from the vein, the spectral Doppler signal corresponding to the section including the vein may be determined as an artifact signal. Thus, the processor 3010 may generate a spectral Doppler signal as a signal obtained by removing the ultrasound reflected signal from the vein from the spectral Doppler signal corresponding to the artery.

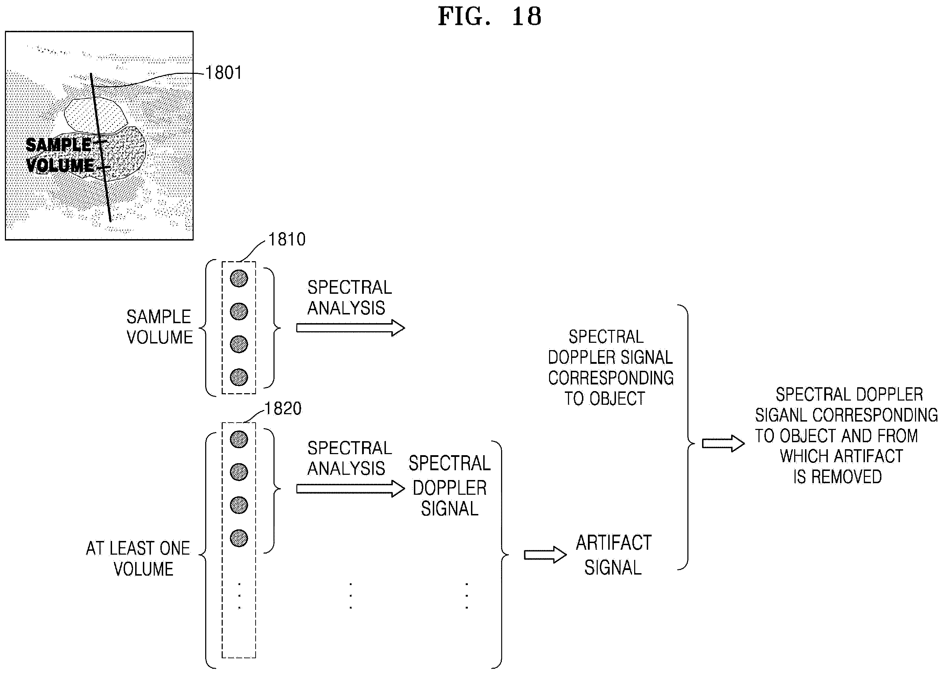

FIG. 18 illustrates another embodiment in which the ultrasonic imaging apparatus 3000 generates a spectral Doppler signal corresponding to an object.

The ultrasonic imaging apparatus 3000 may include the ultrasound transceiver 110.

The ultrasound transceiver 110 may transmit ultrasound signals along a pulse Doppler line 1801 passing through a sample volume set with respect to an object and receive echo signals reflected from the sample volume and at least one volume on the pulse Doppler line 1801. The processor 3010 may acquire from the received echo signals a Doppler signal 1810 corresponding to the sample volume and at least one Doppler signal 1820 corresponding to the at least one volume.

The processor 3010 may generate a spectral Doppler signal corresponding to the object by performing spectral analysis on the Doppler signal 1810 corresponding to the sample volume. Furthermore, the processor 3010 may generate at least one spectral Doppler signal corresponding to the at least one volume by performing spectral analysis on the at least one Doppler signal 1820 corresponding to the at least one volume.

The processor 3010 may model an artifact signal in the spectral Doppler signal corresponding to the object by using the at least one spectral Doppler signal. The processor 3010 may determine as an artifact signal an ultrasound reflected signal from another object, which is contained in the spectral Doppler signal corresponding to the object. The processor 3010 may determine as an artifact signal a spectral Doppler signal corresponding to a volume including another object from among the at least one volume.

Thus, the processor 3010 may generate, based on the spectral Doppler signal corresponding to the object and the artifact signal, a spectral Doppler signal corresponding to the object and from which an artifact is removed.

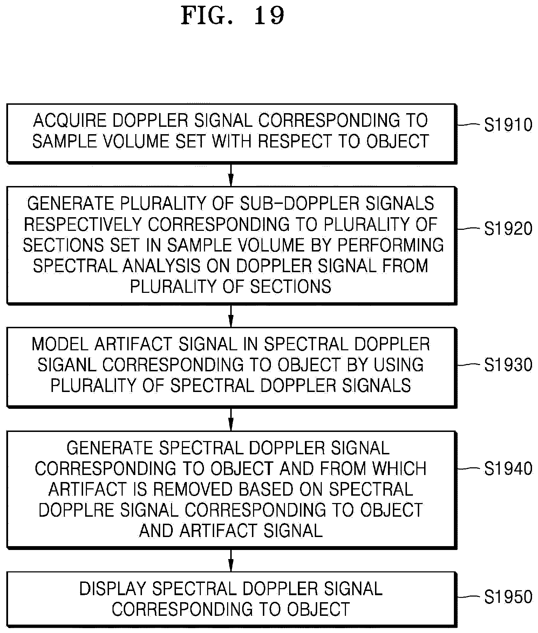

FIG. 19 is a flowchart of a method whereby an ultrasonic imaging apparatus displays an ultrasound image, according to an embodiment.

The method illustrated in FIG. 19 may be performed by each of the components of the ultrasonic imaging apparatus 3000 of FIG. 15, and repeated descriptions with respect to FIG. 15 will be omitted here.

In operation S1910, the ultrasonic imaging apparatus 3000 may acquire a Doppler signal corresponding to a sample volume set with respect to an object. Furthermore, the ultrasonic imaging apparatus may set a plurality of sections in the sample volume.

In operation S1920, the ultrasonic imaging apparatus 3000 may generate a plurality of spectral Doppler signals respectively corresponding to the plurality of sections set in the sample volume by performing a spectral on each of the Doppler signal from the plurality of sections. According to an embodiment, the ultrasonic imaging apparatus 3000 may divide the Doppler signal into a plurality of sub-Doppler signals respectively corresponding to the plurality of sections set in the sample volume and generate a plurality of spectral Doppler signals respectively corresponding to the plurality of sections by performing a plurality of spectral analyses. According to an embodiment, the ultrasonic imaging apparatus 300 may generate a plurality of spectral Doppler signals by performing weighting accumulation and FFT on the respective sub-Doppler signals.

In operation S1930, the ultrasonic imaging apparatus 3000 may model an artifact signal in a spectral Doppler signal corresponding to the object by using the plurality of spectral Doppler signals.

The ultrasonic imaging apparatus 3000 may determine a spectral Doppler signal corresponding to the object from among the plurality of spectral Doppler signals. The ultrasonic imaging apparatus 3000 may determine a spectral Doppler signal corresponding to a section including the object from among the plurality of sections of the sample volume as a spectral Doppler signal corresponding to the object.

The ultrasonic imaging apparatus 3000 may determine an artifact signal in the spectral Doppler signal corresponding to the object. The ultrasonic imaging apparatus 3000 may determine as an artifact signal an ultrasound reflected signal from another object, which is contained in the spectral Doppler signal corresponding to the object. The ultrasonic imaging apparatus 3000 may model an artifact signal by using a spectral Doppler signal corresponding to a section including another object from among the plurality of spectral Doppler signals.

In operation S1940, the ultrasonic imaging apparatus 3000 may generate, based on the spectral Doppler signal corresponding to the object and the artifact signal, a spectral Doppler signal corresponding to the object and from which an artifact is removed. The ultrasonic imaging apparatus 3000 may generate a spectral Doppler signal corresponding to the object and from which an artifact is removed by performing subtraction between the spectral Doppler signal corresponding to the object and the artifact signal.

In operation S1950, the ultrasonic imaging apparatus 3000 may display the spectral Doppler signal corresponding to the object and from which the artifact is removed.

The embodiments may be implemented through computer-readable recording media having stored thereon computer-executable instructions and data. The instructions may be stored in the form of program codes, and when executed by a processor, may generate a predetermined program module to perform a specific operation. Furthermore, when being executed by the processor, the instructions may perform specific operations according to the embodiments.

While embodiments of the disclosure have been particularly shown and described with reference to the accompanying drawings, it will be understood by those of ordinary skill in the art that various changes in form and details may be made therein as far as they fall within the scope of the appended claims. Accordingly, the above embodiments and all aspects thereof are examples only and are not limiting.

**Claims**

1. An ultrasonic imaging apparatus comprising:

   a processor configured to
   acquire a Doppler signal corresponding to a sample volume,
   divide the Doppler signal into a plurality of sub-Doppler signals respectively corresponding to a plurality of sections set in the sample volume.
   generate a plurality of spectral Doppler signals by performing spectral analysis on each of the plurality of sub-Doppler signals,
   determine a spectral Doppler signal corresponding to a section including an object from among the plurality of sections of the sample volume as a spectral Doppler signal corresponding to the object;
   model an artifact signal by using a spectral Doppler signal corresponding to a section including another object from among the plurality of sections of the sample volume; and
   generate a spectral Doppler signal corresponding to the object from which an artifact is removed by performing subtraction between the spectral Doppler signal corresponding to the object and the artifact signal; and
   a display displaying the spectral Doppler signal corresponding to the object from which the artifact is removed.

2. The ultrasonic imaging apparatus of claim 1, wherein the processor is further configured to generate the plurality of spectral Doppler signals by performing weighting accumulation and Fourier Transform on each of the plurality of sub-Doppler signals.

3. The ultrasonic imaging apparatus of claim 1, wherein the processor is further configured to generate the plurality of spectral Doppler signals respectively corresponding to the plurality of sections, and
   wherein the display displays the plurality of spectral Doppler signals.

4. The ultrasonic imaging apparatus of claim 1, wherein the number and length of the plurality of sections are set based on a user input.

5. The ultrasonic imaging apparatus of claim 1, further comprising an ultrasound transceiver configured to control a probe to transmit a generated ultrasound signal to the sample volume as many times as a predetermined ensemble number and according to a preset pulse repetition frequency (PRF), and receive an echo signal reflected from the sample volume,
   wherein the processor is further configured to acquire the Doppler signal based on the echo signal.

6. A method of displaying an ultrasound image, the method comprising:

   acquiring a Doppler signal corresponding to a sample volume;
   dividing the Doppler signal into a plurality of sub-Doppler signals respectively corresponding to a plurality of sections set in the sample volume;
   generating a plurality of spectral Doppler signals by performing spectral analysis on each of the plurality of sub-Doppler signals;
   determining a spectral Doppler signal corresponding to a section including an object from among the plurality of sections of the sample volume as a spectral Doppler signal corresponding to the object;
   modeling an artifact signal by using a spectral Doppler signal corresponding to a section including another object from among the plurality of sections of the sample volume;
   generating a spectral Doppler signal corresponding to the object from which an artifact is removed by performing subtraction between the spectral Doppler signal corresponding to the object and the artifact signal; and
   displaying the spectral Doppler signal corresponding to the object from which the artifact is removed.

7. The method of claim 6, wherein the generating of the plurality of spectral Doppler signals comprises generating the plurality of spectral Doppler signals by performing weighting accumulation and Fourier Transform on each of the plurality of sub-Doppler signals.

8. The method of claim 6, further comprising controlling a probe to transmit a generated ultrasound signal to the sample volume by as many times as a predetermined ensemble number and according to a preset pulse repetition frequency (PRF), and receiving an echo signal reflected from the sample volume,
   wherein the acquiring of the Doppler signal comprises acquiring the Doppler signal based on the echo signal.

9. A computer-readable recording medium having recorded thereon a program for executing the method of claim 6 on a computer of an ultrasonic imaging apparatus.

**Patentansprüche**

1. Ultraschallbildgebungsvorrichtung, die folgendes umfasst:
   einen Prozessor, der zu Folgendem konfiguriert ist:

   Erfassen eines Dopplersignals, das einem Abtastvolumen entspricht,
   Teilen des Dopplersignals in eine Vielzahl von Sub-Dopplersignalen, die jeweils einer Vielzahl von Abschnitten entsprechen, die in dem Abtastvolumen festgelegt sind,
   Erzeugen einer Vielzahl von Spektraldopplersignalen durch Durchführen einer Spektralanalyse an jedem aus der Vielzahl von Sub-Dopplersignalen,
   Bestimmen eines Spektraldopplersignals, das einem Abschnitt aus der Vielzahl von Abschnitten des Abtastvolumens entspricht, der ein Objekt enthält, als ein Spektraldopplersignal, das dem Objekt entspricht,
   Modellieren eines Artefaktsignals unter Verwendung eines Spektraldopplersignals, das einem Abschnitt aus der Vielzahl von Abschnitten des Abtastvolumens entspricht, der ein anderes Objekt enthält, und
   Erzeugen eines dem Objekt entsprechenden Spektraldopplersignals, von dem ein Artefakt entfernt wird, indem eine Subtraktion zwischen dem dem Objekt entsprechenden Spektraldopplersignal und dem Artefaktsignal durchgeführt wird; und
   eine Anzeige, die das dem Objekt entsprechende Spektraldopplersignal anzeigt, von dem das Artefakt entfernt wurde.

2. Ultraschallbildgebungsvorrichtung nach Anspruch 1, wobei der Prozessor ferner so konfiguriert ist, dass er die Vielzahl von Spektraldopplersignalen erzeugt, indem er eine Gewichtungsakkumulation und eine Fourier-Transformation an jedem aus der Vielzahl von Sub-Dopplersignalen durchführt.

3. Ultraschallbildgebungsvorrichtung nach Anspruch 1, wobei der Prozessor ferner so konfiguriert ist, dass er die Vielzahl von Spektraldopplersignalen erzeugt, die jeweils der Vielzahl von Abschnitten entsprechen, und wobei die Anzeige die Vielzahl von Spektraldopplersignalen anzeigt.

4. Ultraschallbildgebungsvorrichtung nach Anspruch 1, wobei die Anzahl und Länge der Vielzahl von Abschnitten basierend auf einer Benutzereingabe eingestellt werden.

5. Ultraschallbildgebungsvorrichtung nach Anspruch 1, ferner umfassend einen Ultraschall-Transceiver, der dazu konfiguriert ist, eine Sonde zu steuern, um ein erzeugtes Ultraschallsignal so oft wie eine vorbestimmte Ensemblezahl und gemäß einer voreingestellten Impulsfolgefrequenz (PRF, Pulse Repetition Frequency) an das Abtastvolumen zu senden, und ein von dem Abtastvolumen reflektiertes Echosignal zu empfangen, wobei der Prozessor ferner dazu konfiguriert ist, das Dopplersignal basierend auf dem Echosignal zu erfassen.

6. Verfahren zum Anzeigen eines Ultraschallbildes, wobei das Verfahren Folgendes umfasst:

Erfassen eines Dopplersignals, das einem Abtastvolumen entspricht;
Teilen des Dopplersignals in eine Vielzahl von Sub-Dopplersignalen, die jeweils einer Vielzahl von Abschnitten entsprechen, die in dem Abtastvolumen festgelegt sind;
Erzeugen einer Vielzahl von Spektraldopplersignalen durch Durchführen einer Spektralanalyse an jedem aus der Vielzahl von Sub-Dopplersignalen,
Bestimmen eines Spektraldopplersignals, das einem Abschnitt aus der Vielzahl von Abschnitten des Abtastvolumens entspricht, der ein Objekt enthält, als ein Spektraldopplersignal, das dem Objekt entspricht;
Modellieren eines Artefaktsignals unter Verwendung eines Spektraldopplersignals, das einem Abschnitt aus der Vielzahl von Abschnitten des Abtastvolumens entspricht, der ein anderes Objekt enthält,
Erzeugen eines dem Objekt entsprechenden Spektraldopplersignals, von dem ein Artefakt entfernt wird, indem eine Subtraktion zwischen dem dem Objekt entsprechenden Spektraldopplersignal und dem Artefaktsignal durchgeführt wird; und
Anzeigen des dem Objekt entsprechenden Spektraldopplersignals, von dem das Artefakt entfernt wurde.

7. Verfahren nach Anspruch 6, wobei das Erzeugen der Vielzahl von Spektraldopplersignalen das Erzeugen der Vielzahl von Spektraldopplersignalen durch Durchführen einer Gewichtungsakkumulation und einer Fourier-Transformation an jedem aus der Vielzahl von Sub-Dopplersignalen umfasst.

8. Verfahren nach Anspruch 6, ferner umfassend das Steuern einer Sonde, um ein erzeugtes Ultraschallsignal so oft wie eine vorbestimmte Ensemblezahl und gemäß einer voreingestellten Impulsfolgefrequenz (PRF, Pulse Repetition Frequency) an das Abtastvolumen zu senden, und das Empfangen eines von dem Abtastvolumen reflektierten Echosignals,
wobei das Erfassen des Dopplersignals das Erfassen des Dopplersignals basierend auf dem Echosignal umfasst.

9. Computerlesbares Aufzeichnungsmedium, auf dem ein Programm zur Ausführung des Verfahrens nach Anspruch 6 auf einem Computer einer Ultraschallbildgebungsvorrichtung gespeichert ist.

**Revendications**

1. Appareil d'imagerie échographique comprenant :
un processeur conçu pour :

acquérir un signal Doppler correspondant à un volume d'échantillonnage,
diviser le signal Doppler en une pluralité de sous-signaux Doppler correspondant respectivement à une pluralité de sections établies dans le volume d'échantillonnage,
générer une pluralité de signaux Doppler spectral, par réalisation d'une analyse spectrale sur chacun des sous-signaux de la pluralité de sous-signaux Doppler,
déterminer un signal Doppler spectral correspondant à une section comportant un objet, parmi la pluralité de sections du volume d'échantillonnage, comme signal Doppler spectral correspondant à l'objet,

modéliser un signal d'artéfact au moyen d'un signal Doppler spectral correspondant à une section comportant un autre objet parmi la pluralité de sections du volume d'échantillonnage, et

générer un signal Doppler spectral correspondant à l'objet, duquel un artéfact est éliminé par réalisation d'une soustraction entre le signal Doppler spectral correspondant à l'objet et le signal d'artéfact ; et

un écran qui affiche le signal Doppler spectral correspondant à l'objet, duquel l'artéfact est éliminé.

2. Appareil d'imagerie échographique selon la revendication 1, dans lequel le processeur est conçu en outre pour générer la pluralité de signaux Doppler spectral par réalisation d'un cumul avec pondération et d'une transformation de Fourier sur chacun des sous-signaux de la pluralité de sous-signaux Doppler.

3. Appareil d'imagerie échographique selon la revendication 1, dans lequel le processeur est conçu en outre pour générer la pluralité de signaux Doppler spectral correspondant respectivement à la pluralité de sections, et dans lequel l'écran affiche la pluralité de signaux Doppler spectral.

4. Appareil d'imagerie échographique selon la revendication 1, dans lequel le nombre et la longueur de la pluralité de sections sont établis compte tenu d'une entrée utilisateur.

5. Appareil d'imagerie échographique selon la revendication 1, comprenant en outre un émetteur-récepteur d'ultrasons conçu pour commander la sonde, pour émettre un signal ultrasonore généré, en direction du volume d'échantillonnage selon un nombre de fois correspondant à un nombre d'ensembles prédéterminé et conformément à une fréquence de répétition d'impulsions (PRF, pulse repetition frequency) préétablie, et pour recevoir un signal d'écho réfléchi par le volume d'échantillonnage,

ledit processeur étant conçu en outre pour acquérir le signal Doppler compte tenu du signal d'écho.

6. Procédé d'affichage d'une image échographique, le procédé comprenant :

l'acquisition d'un signal Doppler correspondant à un volume d'échantillonnage,

la division du signal Doppler en une pluralité de sous-signaux Doppler correspondant respectivement à une pluralité de sections établies dans le volume d'échantillonnage,

la génération d'une pluralité de signaux Doppler spectral, par réalisation d'une analyse spectrale sur chacun des sous-signaux de la pluralité de sous-signaux Doppler,

la détermination d'un signal Doppler spectral correspondant à une section comportant un objet, parmi la pluralité de sections du volume d'échantillonnage, comme signal Doppler spectral correspondant à l'objet,

la modélisation d'un signal d'artéfact au moyen d'un signal Doppler spectral correspondant à une section comportant un autre objet parmi la pluralité de sections du volume d'échantillonnage,

la génération d'un signal Doppler spectral correspondant à l'objet, duquel un artéfact est éliminé, par réalisation d'une soustraction entre le signal Doppler spectral correspondant à l'objet et le signal d'artéfact, et

l'affichage du signal Doppler spectral correspondant à l'objet, duquel l'artéfact est éliminé.

7. Procédé selon la revendication 6, dans lequel la génération de la pluralité de signaux Doppler spectral comprend la génération de la pluralité de signaux Doppler spectral par réalisation d'un cumul avec pondération et d'une transformation de Fourier sur chacun des sous-signaux de la pluralité de sous-signaux Doppler.

8. Procédé selon la revendication 6, comprenant en outre la commande d'une sonde, pour transmettre un signal ultrasonore généré, en direction du volume d'échantillonnage selon un nombre de fois correspondant à un nombre d'ensembles prédéterminé et conformément à une fréquence de répétition d'impulsions (PRF, pulse repetition frequency) préétablie, et la réception d'un signal d'écho réfléchi par le volume d'échantillonnage,

ladite acquisition du signal Doppler comprenant l'acquisition du signal Doppler compte tenu du signal d'écho.

9. Support d'enregistrement lisible par ordinateur sur lequel est enregistré un programme permettant d'exécuter le procédé selon la revendication 6 sur un ordinateur d'un appareil d'imagerie échographique.

# FIG. 1

FIG. 2A

FIG. 2B

FIG. 2C

EP 3 517 043 B1

# FIG. 3

ULTRASONIC IMAGING APPARATUS — 1000

PROCESSOR — 1010

DISPLAY — 1020

# FIG. 4

SPECTRAL DOPPLER SIGNAL CORRESPONDING TO SAMPLE VOLUME

# FIG. 5

EP 3 517 043 B1

# FIG. 6

# FIG. 7

| ACQUIRE DOPPLER SIGNAL CORRESPONDING TO SAMPLE VOLUME | S710 |

| DIVIDE DOPPLER SIGNAL INTO PLURALITY OF SUB-DOPPLER SIGNALS | S720 |

| GENERATE PLURALITY OF SPECTRAL DOPPLER SIGNALS | S730 |

| GENERATE SPECTRAL DOPPLER SIGNAL CORRESPONDING TO SAMPLE VOLUME FROM PLURALITY OF SPECTRAL DOPPLER SIGNALS | S740 |

| DISPLAY SPECTRAL DOPPLER SIGNAL CORRESPONDING TO SAMPLE VOLUME | S750 |

# FIG. 8

ULTRASONIC IMAGING APPARATUS ———2000

PROCESSOR ———2010

DISPLAY ———2020

# FIG. 9

901

FIRST SECTION
SECOND SECTION
:
N-TH SECTION

SAMPLE VOLUME

910

SAMPLE VOLUME

FIRST SECTION

912

SPECTRAL ANALYSIS

FIRST SPECTRAL DOPPLER SIGNAL

SECOND SECTION

914

SPECTRAL ANALYSIS

SECOND SPECTRAL DOPPLER SIGNAL

N-TH SECTION

916

SPECTRAL ANALYSIS

N-TH SPECTRAL DOPPLER SIGNAL

FIG. 10

# FIG. 11

FIRST SPECTRAL DOPPLER SIGNAL

LINE TRACING

FIRST SPECTRAL LINE

SECOND SPECTRAL DOPPLER SIGNAL

LINE TRACING

SECOND SPECTRAL LINE

N-TH SPECTRAL DOPPLER SIGNAL

LINE TRACING

N-TH SPECTRAL LINE

EP 3 517 043 B1

**FIG. 12**

32

FIG. 13

1310

FIRST SECTION    SECOND SECTION    THIRD SECTION    FOURTH SECTION    FIFTH SECTION

SIXTH SECTION    SEVENTH SECTION    EIGHTH SECTION    NINTH SECTION    TENTH SECTION

# FIG. 14

| | |
|---|---|
| ACQUIRE DOPPLER SIGNAL CORRESPONDING TO SAMPLE VOLUME | S1410 |
| DIVIDE DOPPLER SIGNAL INTO PLURALITY OF SUB-DOPPLER SIGNALS | S1420 |
| GENERATE PLURALITY OF SPECTRAL DOPPLER SIGNALS BY RESPECTIVELY PERFORMING SPECTRAL ANALYSES ON PLURALITY OF SUB-DOPPLER SIGNALS | S1430 |
| DISPLAY PLURALITY OF SPECTRAL DOPPLER SIGNALS | S1440 |

FIG. 15

ULTRASONIC IMAGING
APPARATUS ⌐3000

PROCESSOR ⌐3010

DISPLAY ⌐3020

## FIG. 16

SAMPLE VOLUME ⎯1601

SAMPLE VOLUME 1610

1612
SPECTRAL ANALYSIS → SPECTRAL DOPPLER SIGNAL

1614
SPECTRAL ANALYSIS → SPECTRAL DOPPLER SIGNAL

1616
SPECTRAL ANALYSIS → SPECTRAL DOPPLER SIGNAL

SPECTRAL DOPPLER SIGNAL CORRESPONDING TO OBJECT

ARTIFACT SIGNAL

SPECTRAL DOPPLER SIGANL CORRESPONDING TO OBJECT AND FROM WHICH ARTIFACT IS REMOVED

EP 3 517 043 B1

FIG. 17

FIG. 18

# FIG. 19

ACQUIRE DOPPLER SIGNAL CORRESPONDING TO
SAMPLE VOLUME SET WITH RESPECT TO OBJECT ──S1910

GENERATE PLURALITY OF SUB-DOPPLER SIGNALS
RESPECTIVELY CORRESPONDING TO PLURALITY OF
SECTIONS SET IN SAMPLE VOLUME BY PERFORMING ──S1920
SPECTRAL ANALYSIS ON DOPPLER SIGNAL FROM
PLURALITY OF SECTIONS

MODEL ARTIFACT SIGNAL IN SPECTRAL DOPPLER
SIGANL CORRESPONDING TO OBJECT BY USING ──S1930
PLURALITY OF SPECTRAL DOPPLER SIGNALS

GENERATE SPECTRAL DOPPLER SIGNAL
CORRESPONDING TO OBJECT AND FROM WHICH
ARTIFACT IS REMOVED BASED ON SPECTRAL ──S1940
DOPPLRE SIGNAL CORRESPONDING TO OBJECT
AND ARTIFACT SIGNAL

DISPLAY SPECTRAL DOPPLER SIGNAL
CORRESPONDING TO OBJECT ──S1950

**EP 3 517 043 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003163044 A
- US 2013006111 A